(19) **Europäisches Patentamt European Patent Office Office européen des brevets**

(11) **EP 4 269 379 B1**

(12) **EUROPEAN PATENT SPECIFICATION**

(45) Date of publication and mention of the grant of the patent:
**06.08.2025 Bulletin 2025/32**

(21) Application number: **21915226.1**

(22) Date of filing: **24.12.2021**

(51) International Patent Classification (IPC):
C07C 39/16 (2006.01)   C08G 64/20 (2006.01)
C08G 64/06 (2006.01)   C08J 11/14 (2006.01)
C08J 11/24 (2006.01)   C07C 37/14 (2006.01)
C07C 37/20 (2006.01)   C07C 37/52 (2006.01)
C07C 37/74 (2006.01)   C07C 37/84 (2006.01)
C07B 61/00 (2006.01)   C07C 37/055 (2006.01)
C07C 39/04 (2006.01)   C07C 39/19 (2006.01)
C07C 45/41 (2006.01)   C07C 49/08 (2006.01)

(52) Cooperative Patent Classification (CPC):
(C-Sets available)
C07C 37/0555; C07C 37/20; C07C 37/52;
C07C 37/74; C07C 37/84; C07C 45/41;
C08G 64/06; C08J 11/14; C08J 11/24;
C08J 2369/00; Y02P 20/54; Y02W 30/62      (Cont.)

(86) International application number:
**PCT/JP2021/048227**

(87) International publication number:
**WO 2022/145366 (07.07.2022 Gazette 2022/27)**

(54) **METHOD FOR PRODUCING BISPHENOL A AND METHOD FOR PRODUCING POLYCARBONATE RESIN**

VERFAHREN ZUR HERSTELLUNG VON BISPHENOL A UND VERFAHREN ZUR HERSTELLUNG VON POLYCARBONATHARZ

PROCÉDÉ DE PRODUCTION DE BISPHÉNOL A ET PROCÉDÉ DE PRODUCTION DE RÉSINE DE POLYCARBONATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2020 JP 2020218277**

(43) Date of publication of application:
**01.11.2023 Bulletin 2023/44**

(73) Proprietor: **Mitsubishi Chemical Corporation**
**Chiyoda-ku**
**Tokyo 100-8251 (JP)**

(72) Inventors:
• **UCHIYAMA, Kei**
**Tokyo 100-8251 (JP)**
• **HATAKEYAMA, Kazuhisa**
**Tokyo 100-8251 (JP)**

(74) Representative: **Vossius & Partner**
**Patentanwälte Rechtsanwälte mbB**
**Siebertstraße 3**
**81675 München (DE)**

(56) References cited:
WO-A1-2005/077515    JP-A- 2001 302 573
JP-A- 2005 112 781    JP-A- 2005 330 188
JP-A- 2009 242 316    JP-A- 2014 037 368
JP-A- H05 331 088    US-A- 5 198 591

• **JEUNG GON KIM: "Chemical recycling of poly(bisphenol A carbonate)", POLYMER CHEMISTRY, vol. 11, no. 30, 5 August 2020 (2020-08-05), Cambridge, pages 4830 - 4849, XP093162553, ISSN: 1759-9954, DOI: 10.1039/ C9PY01927H**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(Cont. next page)

(52) Cooperative Patent Classification (CPC): (Cont.)

C-Sets
**C07C 37/0555, C07C 39/16;**
**C07C 37/20, C07C 39/16;**

**C07C 37/52, C07C 39/04;**
**C07C 37/52, C07C 39/19;**
**C07C 37/74, C07C 39/16;**
**C07C 37/84, C07C 39/16;**
**C07C 45/41, C07C 49/08**

## Description

Technical Field

The present invention relates to a method for producing bisphenol A. Further, the present invention relates to a method for producing a polycarbonate resin by using bisphenol A obtained by the method for producing bisphenol A.

Background Art

A plastic is easy to use, durable, and inexpensive, and thus is mass-produced not only in Japan but also all over the world. Many of such plastics are used as "disposable" ones and thus some thereof are not appropriately treated and released into the environment. Specifically, a plastic waste flows from a river into the sea and deteriorates because of a wave or an ultraviolet ray in the process to become 5 mm or less in size. Such a small plastic waste is referred to as a microplastic. An animal or fish accidentally swallows this microplastic. As described above, a plastic waste has a tremendous influence on the ecosystem, and, in recent years, has been regarded as a marine plastic problem all over the world. A polycarbonate resin is used in a wide range of fields because of the transparency, mechanical physical properties, flame retardancy, dimensional stability, and electrical characteristics thereof, and this polycarbonate resin is also no exception.

Chemical recycling involving chemically degrading a polycarbonate resin back to a bisphenol for reuse is known as one of the methods for recycling a polycarbonate resin. Chemical recycling of a polycarbonate resin is important as one of the solutions to the marine plastic problem. A polycarbonate resin can be degraded by various methods such as hydrolysis and alcoholysis, and bisphenol A generated can be recovered by crystallization or the like.

It is known that high-purity bisphenol A can be obtained by incorporating bisphenol A obtained by degrading a polycarbonate resin into the following common production process for bisphenol A and purifying together.

[Common production process for bisphenol A]

Step 1: a step of reacting acetone and phenol in the presence of an acidic catalyst to obtain a reaction liquid containing bisphenol A
Step 2: a step of subjecting the reaction liquid obtained in the step 1 to distillation separation to obtain a concentrated liquid
Step 3: a step of subjecting the concentrated liquid obtained in the step 2 to crystallization/recovery to obtain an adduct crystal and a mother liquor Step 4: a step of producing bisphenol A from the adduct crystal

For example, a method is known in which a crude solution containing low-purity bisphenol A recovered from degradation products obtained by pyrolysis or chemical degradation of a waste plastic is supplied to a concentrated liquid or mother liquor obtained by the common production process for bisphenol A (Patent Literature 1).

In addition, a method is known in which a waste polycarbonate is decomposed into isopropenylphenol or the like, which is then supplied to the step 1 (reaction step) of the common production process for bisphenol A (Patent Literature 2).

Patent Literature 3 discloses a process for obtaining bisphenol from a post-reaction mixture resulting from the step of synthesis of phenol and acetone in the presence of a strong acid cation exhanger catalyst, by the way of crystallation and separation by distillation followed by recovery of bisphenol A from the step of thermal catalytic decomposition of a by-product of the principal process technology in a multistage process.

Citation List

Patent Literature

Patent Literature 1: Japanese Patent Laid-Open No. 2005-112781
Patent Literature 2: Japanese Patent Laid-Open No. 2006-36668
Patent Literature 3: US 5,198,591 A

Summary of Invention

# EP 4 269 379 B1

Technical Problem

In some fields, bisphenol A is used as a raw material for optical materials such as optical polycarbonate resins. Optical materials are required to have excellent color tone (transparency), and hence bisphenol A, which is to be used as the raw material, is also required to have excellent color tone.

In the method in which a polycarbonate resin is subjected to hydrolysis or alcoholysis followed by crystallization, however, a slight amount of residual coloring components derived from the polycarbonate resin may lead to insufficiency as a raw material for optical materials, for which excellent color tone (transparency) is required, even if the resulting bisphenol A has high purity. Methods in which the steps from degradation of a polycarbonate resin to purification of bisphenol A are independently carried out need a larger number of steps and more complex or larger-scale facilities to achieve higher purity of bisphenol A, thus being unfavorable in terms of cost and energy.

As described above, high-purity bisphenol A can be obtained also by supplying bisphenol A obtained by degrading a polycarbonate resin to the common production process for bisphenol A and purifying together. According to the description in Example 1 in Patent Literature 1, for example, a compact disk composed of a polycarbonate resin is subjected to alcoholysis with cyclohexanol, and distillation is carried out under reduced pressure, giving a heavy component that was not distilled out; thereafter, the heavy component that was not distilled out is mixed in a mother liquor obtained by crystallization in the typical production process for bisphenol A, the resultant is further mixed with a bisphenol A-containing reaction product obtained by the condensation reaction of the typical production process for bisphenol A, and the resultant is concentrated; after the resulting concentrated liquid is subjected to crystallization, solid-liquid separation is carried out to obtain a bisphenol A-phenol adduct crystal, and phenol is distilled off from the resulting adduct crystal to give high-purity bisphenol A. The resulting bisphenol A has high purity, but disadvantageously suffers from deteriorated color tone because of the influence of coloring components contained in the heavy component that was not distilled out.

Another possible method is alkaline degradation of a polycarbonate resin into isopropenylphenol. According to the description in Example 1 in Patent Literature 2, for example, a polycarbonate resin is degraded into isopropenylphenol by a known method, and the resulting degraded liquid is supplied to a BPA synthesis step of synthesizing bisphenol A; a synthesized liquid obtained through the BPA synthesis step is supplied to the concentration step in the production process for bisphenol A, and a conventional method is applied to give high-purity bisphenol A. However, the isopropenylphenol is a very unstable chemical species, and disadvantageously condenses with various components before supplying the degraded liquid to the bisphenol A synthesis step of synthesizing bisphenol A in the production process. Moreover, the condensed components disadvantageously deteriorate the color tone of bisphenol A to be obtained.

Thus, the conventional methods of chemical recycling of a polycarbonate resin need much time for purification of recycled bisphenol A, and require removal of coloring components derived from a polycarbonate resin and improvement in the color tone of bisphenol A to be obtained, and hence these methods are required to be further improved.

The present invention has been made in view of such circumstances, and an object thereof is to provide a method for producing bisphenol A that can efficiently remove coloring components derived from a polycarbonate resin to enable production of bisphenol A having good color tone. In addition, another object of the present invention is to provide a method for producing a polycarbonate resin by using the obtained bisphenol.

Solution to Problem

The present inventors have carried out a diligent study in order to solve the above problems and, as a result, found a method for producing bisphenol A in which a polycarbonate resin is degraded with phenol, part of phenol is distilled off after degradation to obtain a crude solution containing low-purity bisphenol A, the crude solution and a mother liquor obtained in a production process for bisphenol A are mixed, treatment or the like is then carried out under conditions that allow degradation of bisphenol A, and the obtained reaction liquid is circulated to the production process for bisphenol A. In addition, the present inventors have found a method for producing a polycarbonate resin by using the obtained bisphenol A.

**The present invention is as described in the appended claims.**

Advantageous Effects of Invention

According to the present invention, a method for producing bisphenol A that can efficiently remove coloring components derived from a polycarbonate resin to enable production of bisphenol A having good color tone is provided. In addition, a method for producing a polycarbonate resin by using the obtained bisphenol A is provided.

Brief Description of Drawings

[Figure 1] Figure 1 shows a flow diagram for showing an example of the method of the present invention for producing bisphenol A.
[Figure 2] Figure 2 shows a flow diagram for showing an example of the method of the present invention for producing bisphenol A.
[Figure 3] Figure 3 shows a flow diagram for showing an example of a step A of the method of the present invention for producing bisphenol A.
[Figure 4] Figure 4 shows a flow diagram for showing an example of a step A of the method of the present invention for producing bisphenol A.
[Figure 5] Figure 5 shows a flow diagram for showing an example of the method of the present invention for producing bisphenol A.
[Figure 6] Figure 6 shows a flow diagram for showing an example of a step H of the method of the present invention for producing bisphenol A.
[Figure 7] Figure 7 shows a flow diagram for showing an example of a step H of the method of the present invention for producing bisphenol A.
[Figure 8] Figure 8 shows a flow diagram for showing an example of a step H of the method of the present invention for producing bisphenol A.
[Figure 9] Figure 9 shows a flow diagram for showing an example of a step H of the method of the present invention for producing bisphenol A.
[Figure 10] Figure 10 shows a flow diagram for showing an example of a step H of the method of the present invention for producing bisphenol A.

Description of Embodiment

Hereinafter, an embodiment of the present invention will be described in detail, but the description of the constituent requirements described below is an example of the embodiment of the present invention, and the present invention is not limited to the contents of the following description as long as they do not depart from the scope of the present invention. In addition, as used herein, an expression of a range including the term "to" with numerical values or physical property values provided before and after the term is used to mean that the values before and after the term are included in the range.

<Method for producing bisphenol A>

The present invention relates to a method for producing bisphenol A, including the following step A to step F, step H, and step I (hereinafter, sometimes referred to as the "method of the present invention for producing bisphenol A").

Step A: a step of degrading a polycarbonate resin in a solvent to obtain a reaction liquid a1 containing bisphenol A, and distilling off the solvent from the obtained reaction liquid a1 to obtain a crude solution A having a bisphenol A content of less than 90% by mass
Step B: a step of subjecting acetone and phenol to dehydration condensation in the presence of an acid catalyst to obtain a reaction liquid B containing bisphenol A
Step C: a step of distilling off unreacted acetone and water from the reaction liquid B obtained in the step B to obtain a concentrated liquid C
Step D: a step of subjecting the concentrated liquid C obtained in the step C to crystallization to obtain a slurry liquid, and subjecting the slurry liquid to solid-liquid separation to obtain a mother liquor D and a cake d
Step E: a step of purifying the cake d obtained in the step D to obtain bisphenol A
Step F: a step of circulating part of the mother liquor D obtained in the step D to supply to the step B
Step H: any step of the following (I) to (III)

(I) a step of obtaining a solution H1 or a solution H2 from the crude solution A and part of the mother liquor D The solution H1 in (I) is a solution containing bisphenol A obtained by degrading bisphenol A contained in the crude solution A and the mother liquor D into phenol and isopropenylphenol under conditions that allow degradation of bisphenol A and then rebonding phenol and isopropenylphenol. The solution H2 in (I) is a solution obtained by degrading bisphenol A contained in the crude solution A and the mother liquor D into phenol and acetone under conditions that allow degradation of bisphenol A.

(II) a step of subjecting part of the mother liquor D to isomerization treatment and crystallization/solid-liquid separation treatment and obtaining a solution H1 or a solution H2 from a mother liquor S2a obtained and the crude solution A

The solution H1 in (II) is a solution containing bisphenol A obtained by degrading bisphenol A contained in the mother liquor S2a and the crude solution A into phenol and isopropenylphenol under conditions that allow degradation of bisphenol A and then rebonding phenol and isopropenylphenol. The solution H2 in (II) is a solution obtained by degrading bisphenol A contained in the mother liquor S2a and the crude solution A into phenol and acetone under conditions that allow degradation of bisphenol A.

(III) a step of subjecting a solution S3b containing a solution S3a after isomerization treatment of the mother liquor D and the crude solution A to crystallization/solid-liquid separation treatment and obtaining a solution H1 or a solution H2 from a mother liquor S2b obtained

The solution H1 in (III) is a solution containing bisphenol A obtained by degrading bisphenol A contained in the mother liquor S2b into phenol and isopropenylphenol under conditions that allow degradation of bisphenol A and then rebonding phenol and isopropenylphenol. The solution H2 in (III) is a solution obtained by degrading bisphenol A contained in the mother liquor S2b into phenol and acetone under conditions that allow degradation of bisphenol A.

Step I: a step of supplying the solution H1 or the solution H2 obtained in the step H to the step B and/or the step C

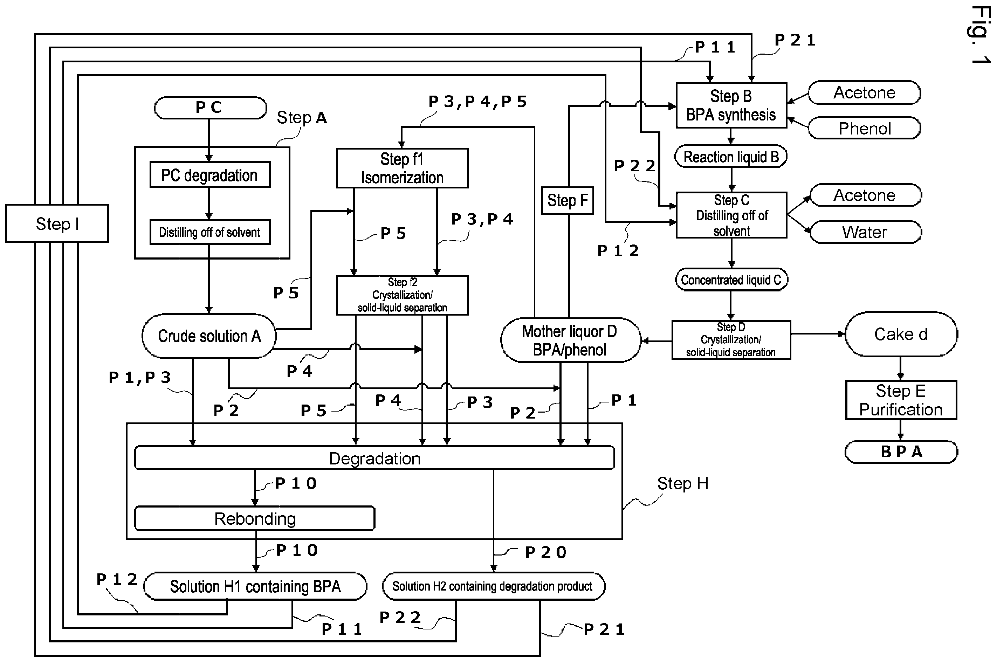

Figure 1 shows a flow diagram for showing an example of the method of the present invention for producing bisphenol A. As illustrated in Figure 1, bisphenol A generated through degradation of a polycarbonate resin is subjected to the step H or the step f2. In the case that the crude solution A and the mother liquor D are treated in the step H, the crude solution A and the mother liquor D are passed through the pathway P1 or P2 in Figure 1 and supplied to an apparatus that carries out degradation in the step H. In the case that the crude solution A and the mother liquor S2a are treated in the step H, the crude solution A and the mother liquor S2a are passed through the pathway P3 or P4 in Figure 1 and supplied to an apparatus that carries out degradation in the step H. In the case that the mother liquor S2b is treated in the step H, the crude solution A and the mother liquor D are passed through the pathway P5 in Figure 1 and supplied to an apparatus that carries out degradation in the step H.

The solution H1 obtained in the step H is sent to the step I to be subjected to the step B (pathway P11 in Figure 1) and/or the step C (pathway P12 in Figure 1). The solution H2 obtained in the step H is subjected to the step B (pathway P21 in Figure 1) and/or the step C (pathway P22 in Figure 1).

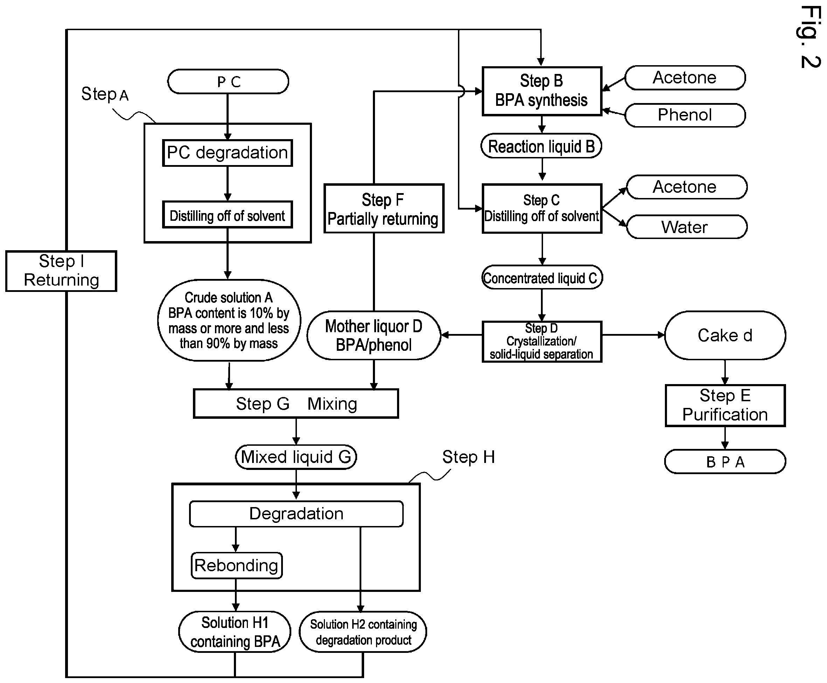

Figure 2 shows a flow diagram for showing an example of the method of the present invention for producing bisphenol A. The method shown in Figure 2 for producing bisphenol A is an example including the following step A to step I in which the crude solution A and part of the mother liquor D are mixed and treated.

Step A: a step of degrading a polycarbonate resin in a solvent to obtain a reaction liquid a1 containing bisphenol A, and distilling off the solvent from the obtained reaction liquid a1 to obtain a crude solution A having a bisphenol A content of less than 90% by mass

Step B: a step of subjecting acetone and phenol to dehydration condensation in the presence of an acid catalyst to obtain a reaction liquid B containing bisphenol A

Step C: a step of distilling off unreacted acetone and water from the reaction liquid B obtained in the step B to obtain a concentrated liquid C

Step D: a step of subjecting the concentrated liquid C obtained in the step C to crystallization to obtain a slurry liquid, and subjecting the slurry liquid to solid-liquid separation to obtain a mother liquor D and a cake d

Step E: a step of purifying the cake d obtained in the step D to obtain bisphenol A

Step F: a step of circulating part of the mother liquor D obtained in the step D to supply to the step B

Step G: a step of mixing the crude solution A obtained in the step A and part of the mother liquor D obtained in the step D to obtain a mixed liquid G

Step H: a step of obtaining a solution H1 containing bisphenol A, wherein the solution H1 is obtained by treating the mixed liquid G under conditions that allow degradation of bisphenol A and then rebonding, or a solution H2 containing a degradation product, wherein the solution H2 is obtained by treating the mixed liquid G under conditions that allow degradation of bisphenol A

Step I: a step of supplying the solution H1 or the solution H2 obtained in the step H to the step B and/or the step C

One of the features of the method of the present invention for producing bisphenol A, shown in Figure 2, is that a crude solution A obtained by degrading a polycarbonate resin is mixed with a mother liquor D obtained through production of bisphenol A, and the resultant is subjected to treatments including degradation and rebonding in the step H and then returned to the step of generating bisphenol A (step B) and/or the step of concentrating the reaction liquid B (step C). The reaction liquid obtained by degrading a waste plastic containing a polycarbonate resin may contain a stabilizer derived from the degraded polycarbonate resin, resins other than the polycarbonate resin, sebum, dusts, foreign substances, and others. Conventionally, such a reaction liquid is supplied to a step of reacting acetone and phenol in the presence of an acidic catalyst to obtain a reaction liquid containing bisphenol A, a step of subjecting the reaction liquid containing bisphenol A to distillation separation to obtain a concentrated liquid, and a step of subjecting the concentrated liquid to crystallization/recovery to obtain an adduct crystal and a mother liquor in the common production process for bisphenol A, which causes significant contamination in such production processes for bisphenol A, and bisphenol A produced may have deteriorated quality.

On the other hand, some production processes for bisphenol A include a step of circulating a mother liquor. The mother liquor to be circulated to the step of subjecting acetone and phenol to dehydration condensation to generate bisphenol A is subjected to recovery of useful components in the mother liquor or treated to reduce impurities before being circulated to the step.

The crude solution A obtained in the step A usually contains not only bisphenol A but also impurities of heavy components that are derived from degradation products of the polycarbonate resin and the structures of which have not been analyzed well. Heavy components are components having higher boiling point than phenol. The impurities of heavy components are different from impurities by-produced in production of bisphenol A, and it is not always possible to remove the impurities of heavy components by the same purification method as in the method for producing bisphenol A. Therefore, in the case that the crude solution A is used for the common production process for bisphenol A, the step of supplying the crude solution A, if the step is carried out in an improper manner, may lower the purity of or deteriorate the color tone of bisphenol A to be obtained. The present inventors have found that useful components in the crude solution A obtained by degrading the polycarbonate resin can be recovered and impurities can be degraded or removed under treatment conditions for recovering useful components in the mother liquor and under treatment conditions for reducing impurities, respectively. Moreover, the present inventors have found that high-purity bisphenol A having excellent color tone can be obtained by incorporating the crude solution A into the step of circulating the mother liquor among the steps of the production process for bisphenol A. Specifically, not only bisphenol A in the crude solution A but also residual incomplete degradation products of the polycarbonate resin (e.g., multimers, such as dimers and trimers, of bisphenol A) are degraded and coloring factors are removed by carrying out the step H, which involves mixing the crude solution A with the mother liquor D and treating under conditions that allow degradation of bisphenol A, and the treated solution is then returned to the step B and/or the step C to give bisphenol A having excellent color tone in the purification step E.

One of the features of the method of the present invention for producing bisphenol A is that a crude solution A having a bisphenol content of less than 90% by mass is prepared in the step A. Because not only bisphenol A in the crude solution A but also residual incomplete degradation products are degraded in the step H, it is not needed to carry out excessive purification operations in the step A; thus the step A can be simplified. Excessively high bisphenol A content imparts high viscosity to the crude solution A, complicating transfer of the crude solution A in order to mix with the mother liquor D obtained in the step D, and making it difficult to homogenously mix with the mother liquor D obtained in the step D. Excessively low bisphenol A content leads to a smaller amount of bisphenol A to be produced, which is economically unfavorable.

Next, the steps of the method of the present invention for producing bisphenol A will be described.

[Step A]

The step A is a step of degrading a polycarbonate resin in a solvent to obtain a reaction liquid a1 containing bisphenol A, and distilling off the solvent from the obtained reaction liquid a1 to obtain a crude solution A having a bisphenol A content of less than 90% by mass.

(Polycarbonate resin (PC))

Polycarbonate resins applicable to the step A includes a polycarbonate resin containing a repeating unit derived from bisphenol A (2,2-bis(4-hydroxyphenyl)propane).

As the polycarbonate resin, not only a polycarbonate resin containing a repeating unit derived from bisphenol A alone but also a composition including a resin other than a polycarbonate resin such as a copolymer or a polymer alloy may be used. Examples of the composition including a resin other than a polycarbonate resin include a polycarbonate/polyester copolymer, a polycarbonate/polyester alloy, a polycarbonate/polyarylate copolymer, and a polycarbonate/polyarylate alloy. When a composition including a resin other than a polycarbonate resin is used, a composition including a polycarbonate resin as the main component (the composition includes 50% by mass or more of a polycarbonate resin) is suitable.

In addition, as the polycarbonate resin, two or more different polycarbonate resins may be mixed and used.

From the viewpoint of chemical recycling, the polycarbonate resin is preferably a polycarbonate resin included in a waste plastic. The polycarbonate resin is molded into various molded articles such as an optical member such as a headlamp or an optical recording medium such as an optical disk and used. As the waste plastic including a polycarbonate resin, a scrap or a defective product when molding and processing the polycarbonate resin into such a molded article, a used molded article, or the like can be used.

The waste plastic may be appropriately washed, crushed, ground, or the like before use. The methods for crushing the waste plastic include coarse crushing using a jaw crusher or a gyratory crusher for crushing to 20 cm or less, medium crushing using a gyratory crusher, a corn crusher, or a mill for crushing to 1 cm or less, grinding using a mill for crushing to 1 mm or less, or the like, and may be any method that can reduce the waste plastic to a size at which it can be supplied to a degradation tank. In addition, when the waste plastic is a thin plastic such as a CD or a DVD, the thin plastic can be cut by using a shredder or the like and supplied to a degradation tank. In addition, another resin of a copolymer or a polymer alloy, or a portion formed of a component other than a polycarbonate resin such as a layer on the front surface or the back surface of an optical disk may be removed in advance before use.

(Degradation of polycarbonate resin)

Known methods can be used for degrading the polycarbonate resin. For example, heating the polycarbonate resin in a solvent causes degradation of the polycarbonate resin, giving a reaction liquid a1 containing bisphenol A.

Especially, use of phenol for degradation of the polycarbonate resin is preferable because the dissolution rate of the polycarbonate resin is high. That is, it is preferable to degrade the polycarbonate resin in a solvent containing phenol.

The solvent containing phenol may contain a solvent other than phenol, but preferably contains phenol as the main component. For example, the mass of phenol in the solvent containing phenol is preferably 50% by mass or more, and can be appropriately set to, for example, 65% by mass or more, 80% by mass or more, 85% by mass or more, or 90% by mass or more according to the type of the additional solvent or a catalyst.

The solvent containing phenol is preferably a mixed solvent containing any solvent selected from the group consisting of water, a monohydric alcohol, and a dihydric alcohol and phenol, and more preferably a mixed solvent containing phenol and water or a mixed solvent containing phenol and a monohydric alcohol, because the degradation rate of the polycarbonate resin is higher than that in the case of using phenol alone as the solvent and the polycarbonate resin can be degraded even under mild conditions (e.g., at normal pressure and about 60 to 150°C). The monohydric alcohol is preferably a linear alcohol having 1 to 5 carbon atoms such as methanol, ethanol, and n-butanol.

It is preferable to use a catalyst for degradation of the polycarbonate resin, and it is preferable to use any catalyst selected from the group consisting of an alkali metal hydroxide, an alkali metal carbonate, an alkylamine, and an acid.

The alkali metal hydroxide is preferably sodium hydroxide or potassium hydroxide. The alkali metal carbonate is preferably sodium carbonate or potassium carbonate.

The alkylamine is a compound obtained by replacing at least one hydrogen atom of ammonia with an alkyl group. Examples of the alkylamine include methylamine, ethylamine, propylamine, dimethylamine, diethylamine, trimethylamine, and triethylamine. The alkylamine is preferably a secondary amine or a tertiary amine, and more preferably a tertiary amine.

The boiling point of the alkylamine is preferably 200°C or less, and more preferably 160°C or less. The lower limit is preferably 10°C or more, and more preferably 30°C or more. In distilling off part of the solvent by distillation under reduced pressure or the like, such boiling point allows the alkylamine to be removed together outside the system.

The acid is preferably any selected from the group consisting of hydrochloric acid, sulfuric acid, phosphoric acid, and sulfonic acid. Examples of the sulfonic acid include an alkylsulfonic acid such as methanesulfonic acid and an aromatic sulfonic acid such as toluenesulfonic acid.

The mole ratio of the catalyst to 1 mol of the repeating unit of the polycarbonate resin ((mass [g] of catalyst used / molecular weight [g/mol] of catalyst) / (mass [g] of polycarbonate resin used / molecular weight [g/mol] of repeating unit)) is preferably 0.0001 or more, more preferably 0.0005 or more, and further preferably 0.0007 or more. When the amount of the catalyst used is small based on that of the polycarbonate resin, the degradation rate decreases, the degradation time is lengthened, and the efficiency tends to deteriorate. The mole ratio of the catalyst to 1 mol of the repeating unit of the polycarbonate resin is preferably 1 mol or less, more preferably 0.9 or less, and further preferably 0.8 or less. When the amount of the catalyst used is large based on that of the polycarbonate resin used, the production efficiency tends to decrease.

(Distilling off of solvent)

In the step A, after degrading the polycarbonate resin, the solvent is distilled off from the obtained reaction liquid a1. Distilling off of the solvent can be carried out by distillation. The solvent in the reaction liquid a1 is not needed to be completely distilled off by distillation, and the amount of the solvent to be distilled off is appropriately determined, for example, according to the bisphenol A content intended and components contained in the reaction liquid a1.

When a solvent containing phenol is used, it is preferable to distil off part of phenol after degrading the polycarbonate resin in the presence of phenol in order to efficiently supply bisphenol A obtained by degrading the polycarbonate resin, for example, to the step G or the step H. If a light component is contained in the crude solution A, the light component may remain in the solution H1 or the solution H2 obtained in the step H to lower the quality of bisphenol A obtained in the step H. Removal of part of phenol after degrading the polycarbonate resin in the presence of phenol in the step A gives a crude solution A sufficiently removed of light components. A light component refers to a component having lower boiling point than phenol, and when a mixed solvent of phenol and water or an alkylamine or the like as a catalyst is used, for example, water or the alkylamine or the like acts as a light component. When a mixed solvent of phenol and a monohydric alcohol is used, the monohydric alcohol and dialkyl carbonates by-produced together with bisphenol A act as light components.

(Crude solution A)

The crude solution A is a composition containing bisphenol A in a proportion of less than 90% by mass and being liquid under a temperature condition for subjecting to the step G or the step H. The crude solution A may be solid at a temperature lower than a temperature condition for subjecting to the step G or the step H. The crude solution A obtained in the step A is usually transferred at 40°C or more to an apparatus that carries out the step G or the step H, and hence the crude solution A is a composition containing bisphenol A and being liquid at 40°C or more.

The bisphenol A content of the crude solution A containing bisphenol A (mass of bisphenol A / mass of crude solution A $\times$ 100 (%)), which is obtained in the step A, is less than 90% by mass. The bisphenol A content is preferably 85% by mass or less, and more preferably 80% by mass or less, 70% by mass or less, or 60% by mass or less, where a smaller value is more preferable. As described above, even when the crude solution A contains impurities of heavy components such as incomplete degradation products of the polycarbonate resin (e.g., multimers, such as dimers and trimers, of bisphenol A) in the method of the present invention for producing bisphenol A, the impurities of heavy components can be degraded or removed in the step H, and hence excessive purification is not needed for the crude solution A. Excessively high bisphenol A content imparts high viscosity to the crude solution A, complicating transfer of the crude solution A and making it difficult to homogenously mix with the mother liquor D obtained in the step D. In addition, if the bisphenol A content is excessively high, in rebonding phenol and isopropenylphenol generated by degrading bisphenol A in the step H, isopropenylphenol is formed in high concentration through degradation treatment such as alkaline degradation in the step H, and undergoes selfcondensation to generate byproducts, which results in a significantly lowered formation rate of bisphenol A in rebonding.

The bisphenol A content of the crude solution A is preferably 10% by mass or more, and more preferably 20% by mass or more, 30% by mass or more, or 40% by mass or more, where a larger value is more preferable. Excessively low bisphenol A content leads to a smaller amount of bisphenol A to be produced, which is economically unfavorable.

The crude solution A may contain phenol in addition to bisphenol A. The phenol content of the crude solution A is preferably 10% by mass or more, more preferably 15% by mass or more, and particularly preferably 20% by mass or more. Excessively low phenol content causes the precipitation of bisphenol A in the crude solution A to generate slurry liquid, leading to difficulty in supplying.

Although the crude solution A may contain heavy components such as incomplete degradation products of the polycarbonate resin (e.g., multimers, such as dimers and trimers, of bisphenol A), excessively large amounts of heavy components may result in generation of carbon dioxide to cause pressure variation in the step H, leading to complexity in reaction control. Therefore, the heavy component content of the crude solution A is preferably 5% by mass or less, and more preferably 1% by mass or less. The crude solution A may be sufficiently washed.

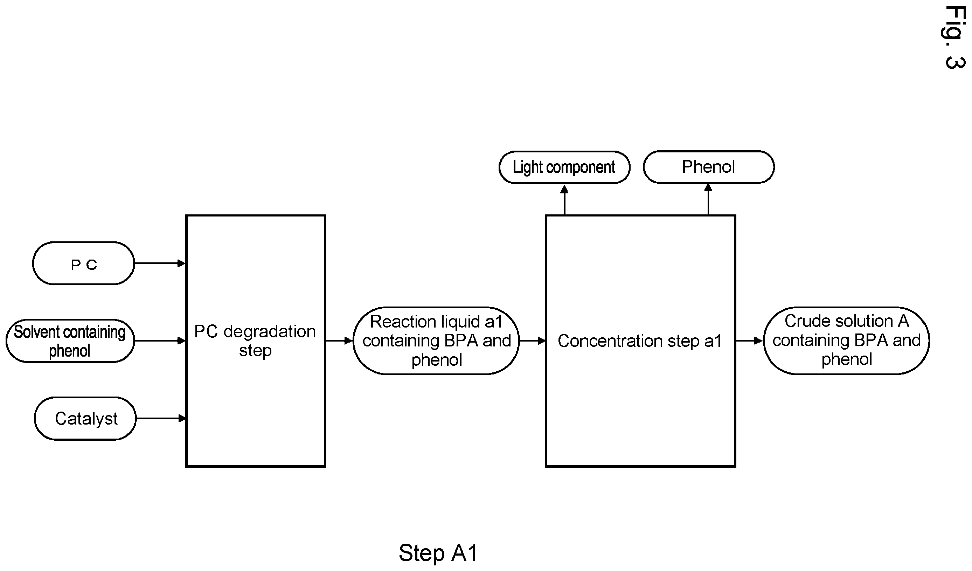

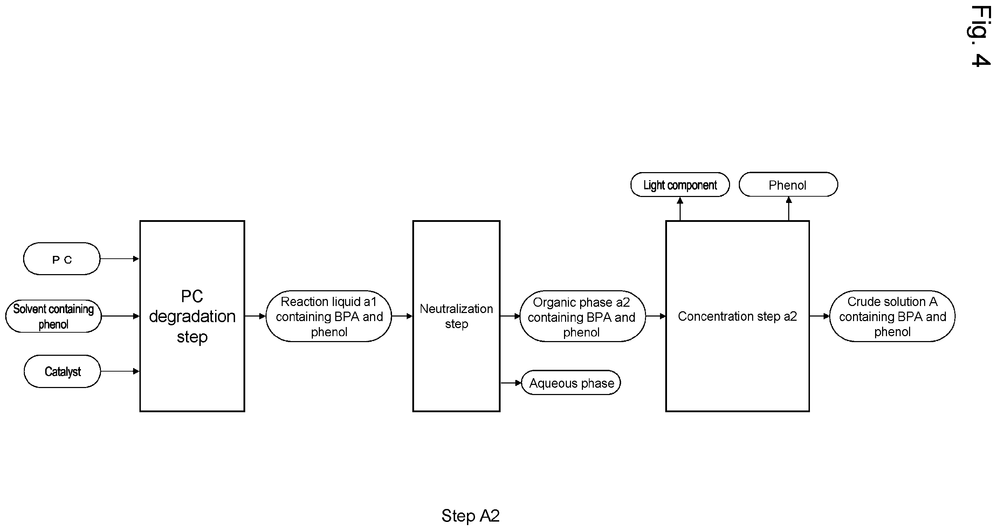

Specific examples of the step A will be described in the following with reference to Figure 3 and Figure 4.

[Step A1]

The step A1 illustrated in Figure 3 includes a PC degradation step of degrading the polycarbonate resin in the presence of a solvent containing phenol and a catalyst to obtain a reaction liquid a1 containing bisphenol A, and a concentration step a1 of subjecting the reaction liquid a1 obtained in the PC degradation step to distillation to distil off part of phenol. In the step A1, a catalyst that can be distilled off in the concentration step a1 is usually used. Examples of such catalysts include an alkylamine.

(PC degradation step)

The reaction temperature is usually 60 to 150°C. The reaction temperature is preferably 70°C or more, more preferably 75°C or more, and further preferably 80°C or more. The upper limit of the reaction temperature can be appropriately set to, for example, 130°C or less, 120°C or less, 110°C or less, 100°C or less, or 95°C or less, according to the type of the solvent used for degradation of the polycarbonate resin. The pressure in the degradation reaction of the PC is preferably 1 kPa to 50 MPa, and more preferably 5 kPa to 10 MPa.

The reaction method for the degradation reaction of the polycarbonate resin is not particularly limited, and may be a continuous method or a batch method. In the case of the batch method, for example, the reaction time is appropriately selected depending on the concentration of the polycarbonate resin, the reaction temperature, and the reaction pressure or the like, and when the reaction time is long, the produced bisphenol A tends to be degraded, and thus the reaction time is preferably 30 hours or less, and more preferably 25 hours or less, 20 hours or less, 15 hours or less, 10 hours or less, or 5 hours or less, where a smaller value is more preferable. In addition, when the reaction time is short, the degradation reaction may not proceed sufficiently, and thus the reaction time is preferably 0.1 hours or more, more preferably 0.5 hours or more, and further preferably 1 hour or more.

(Concentration step a1)

In the concentration step a1, the reaction liquid a1 is subjected to distillation to distill off part of phenol. The obtained solution may contain heavy components such as incomplete degradation products of the polycarbonate resin (e.g., multimers, such as dimers and trimers, of bisphenol A) as described above, but even these heavy components can be degraded or removed in the step H, and thus the obtained solution can be directly used as the crude solution A. For example, distillation can be carried out at a temperature of 50 to 200°C and a pressure of 0.1 kPa to 150 kPa. In addition, light components such as an alkylamine, which have lower boiling point than phenol, are also distilled off in the concentration step a1.

[Step A2]

The step A2 illustrated in Figure 4 includes a PC degradation step of degrading the polycarbonate resin in the presence of a solvent containing phenol and a catalyst to obtain a reaction liquid a1 containing bisphenol A, a neutralization step of neutralizing the reaction liquid a1 obtained in the PC degradation step to obtain an organic phase a2 containing bisphenol A, and a concentration step a2 of subjecting the organic phase a2 obtained in the neutralization step to distillation to distil off part of phenol. In the step A2, the reaction liquid a1 is neutralized and the catalyst and the like are removed, and the solvent is then distilled off. In the step A2, even a catalyst having high boiling point such as an alkali metal hydroxide can be used because the catalyst can be removed in the neutralization step. As the catalyst, any selected from the group consisting of an alkali metal hydroxide, an alkali metal carbonate, an alkylamine, and an acid can be used.

(PC degradation step)

The PC degradation step can be carried out as in the step A1.

(Neutralization step)

In the neutralization step, the reaction liquid a1 obtained in the PC degradation step is neutralized to obtain an organic phase a2 containing bisphenol A. When a base such an alkali metal hydroxide, an alkali metal carbonate, or an alkylamine is used as the catalyst, the neutralization is carried out by mixing an acid such as hydrochloric acid, sulfuric acid, and phosphoric acid in the reaction liquid a1. When an acid is used as the catalyst, the neutralization is carried out by mixing a base such as sodium carbonate and sodium hydroxide in the reaction liquid a1. The neutralization is preferably carried out with adjustment of the amount of an acid or base to be mixed to adjust the pH of the reaction liquid a1 to 7.5 to 10 (preferably, pH 8.0 to 9.5). An acid or base is mixed into the reaction liquid a1 according to the catalyst used, oil water separation thereof is then carried out, and the aqueous phase is removed to obtain an organic phase a2 containing bisphenol A.

(Concentration step a2)

In the concentration step a2, the organic phase a2 obtained in the neutralization step is subjected to distillation to distill off part of phenol. The obtained solution may contain incomplete degradation products of the polycarbonate resin (e.g., multimers, such as dimers and trimers, of bisphenol A) and heavy impurities having higher boiling point than phenol, but even these incomplete degradation products and impurities can be degraded or removed in the step H, and thus the obtained solution can be directly used as the crude solution A. For example, distillation can be carried out at a temperature of 50 to 200°C and a pressure of 0.1 kPa to 150 kPa.

Even in the case of using a catalyst having high boiling point such as an alkali metal hydroxide, if bisphenol A is to be degraded under alkaline conditions in the step H, the crude solution A may be obtained without neutralization as in the step A1.

[Step B]

The step B is a step of subjecting acetone and phenol to dehydration condensation in the presence of an acid catalyst to obtain a reaction liquid B containing bisphenol A.

(Acetone)

Any industrially available product of acetone can be used as a raw material without limitation. For example, acetone may be newly supplied from the outside of the system, unreacted acetone distilled off in the step C described later may be circulated to be used as the acetone in the step B, and these acetones may be mixed for use.

(Phenol)

Any industrially available product of phenol can be used as a raw material without limitation. For example, phenol may be newly supplied from the outside of the system, unreacted phenol distilled off in the step C described later or phenol contained in a solution after the step G may be circulated to be used as the phenol in the step B, and these phenols may be mixed for use.

(Acid catalyst)

An acidic substance is used as the acid catalyst, and a mineral acid such as hydrochloric acid and sulfuric acid or a solid acid such as a highly acidic cation-exchange resin and polysiloxane can be used. From the viewpoints of, for example, corrosion of apparatuses, separation of the catalyst after the reaction, and catalytic activity, a highly acidic cation-exchange resin such as ones of sulfonic acid type is usually used, and, for example, an acidic cation-exchange resin of styrene-divinyl benzene copolymer type formed by introducing sulfonic acid groups in a number of about 2 to 16% of the total number of benzene rings can be used. The average particle size is usually 0.2 to 2 mm, and preferably 0.4 to 1.5 mm. It is preferable for the purpose of improving the selectivity and conversion rate to add a sulfur-containing amine compound as a promoter in the reaction or allow the acid catalyst to support the sulfur-containing amine compound, and examples of acid catalysts suitable for the step B include a highly acidic cation-exchange resin partially modified with a sulfur-containing amine compound.

(Mole ratio between acetone and phenol)

The mole ratio between the raw materials phenol and acetone is not particularly limited, and it is suitable to use phenol in an amount excessively larger than the stoichiometric amount, and 3 to 30 mol, preferably 5 to 20 mol of phenol is used per mol of acetone. If the amount of usage of phenol per mol of acetone is less than 3 mol, the selectivity for bisphenol A is low; if the amount of usage of phenol per mol of acetone is more than 30 mol, problems including lowering of the reaction rate and need of a huge apparatus arise.
For the condensation reaction between acetone and phenol, a known method can be used. The reaction method for the condensation reaction between phenol and acetone is not particularly limited, and the condensation reaction is usually carried out by a fixed-bed flow method or a suspended-bed batch method. In the case of the fixed-bed flow method, the liquid hourly space velocity of the raw material mixture to be supplied to the reactor is usually 0.2 to 50/hours. In the case of the suspended-bed batch method, the amount of usage of the acid catalyst depends on the reaction temperature and the reaction pressure, and is usually 20 to 100% by mass based on the amount of the raw material mixture, and the reaction time is usually 0.5 to 5 hours. Especially, it is preferable to use a fixed-bed continuous reaction method of continuously supplying phenol and acetone for reaction to a condensation reaction apparatus packed with an acidic cation-exchange resin onto which a promoter has been fixed.
The reaction temperature is usually 40 to 130°C, and preferably 40 to 90°C. Reaction temperature of less than 40°C is unpreferable because the reaction liquid may solidify. High temperature over 130°C allows acidic groups of an acidic cation-exchange resin, as the reaction catalyst, to be eliminated from the catalyst and contaminate bisphenol A to cause degradation of bisphenol A, or the high temperature causes degradation of the catalyst to shorten the catalyst lifetime. The reaction pressure is usually normal pressure to 600 kPa (absolute pressure).

[Step C]

The step C is a step of distilling off unreacted acetone and water from the reaction liquid B obtained in the step B to obtain a concentrated liquid C. The reaction liquid B obtained in the step B contains generated bisphenol A, unreacted acetone, phenol, and by-produced water, an isomer of bisphenol A, and others. In the step C, light components including unreacted acetone and water are removed from the reaction liquid B outside the system by a method of, for example, distillation under reduced pressure for the reaction liquid B to obtain a concentrated liquid C containing bisphenol A and phenol.

In the step C, it is preferable to distill off acetone, water, and part of phenol from the reaction liquid B obtained in the step B to obtain a concentrated liquid C. A concentrated liquid C sufficiently removed of acetone and water is obtained by distilling off part of phenol.

For example, the reaction liquid B obtained in the step B is transferred to a distillation column, water, unreacted acetone, and part of phenol are removed from the top of the column, and the reaction product is then withdrawn from the bottom of the column; thus, a concentrated liquid C to be used in the step D can be obtained. The obtained concentrated liquid C is subjected to the step D. The acetone distilled off may be circulated to the step B. The phenol distilled off may be circulated to the step B, or used as a washing solvent for bisphenol A in the step E.

Distillation is preferably carried out at a reaction temperature of 50 to 150°C and a pressure of 0.0065 to 0.040 MPa. Usually, acetone and water in the concentrated liquid C are each preferably removed to 0.1% by mass or less. Thereby, the solubility of an adduct crystal during the crystallization operation is lowered, leading to an improved crystal yield. The concentration of bisphenol A in the concentrated liquid C is preferably 20 to 50% by mass. If the concentration of bisphenol A is less than 20% by mass, a lower yield results; if the concentration of bisphenol A is more than 50% by mass, higher viscosity is imparted to the concentrated liquid C, leading to difficulty in transportation.

[Step D]

The step D is a step of subjecting the concentrated liquid C obtained in the step C to crystallization to obtain a slurry liquid, and subjecting the slurry liquid to solid-liquid separation to obtain a mother liquor D and a cake d.

In the step D, the concentrated liquid C is first subjected to crystallization to precipitate an adduct crystal of bisphenol A and phenol. For example, the concentrated liquid C is transferred to a crystallization apparatus with the temperature adjusted usually to 60 to 100°C, preferably to 70 to 90°C. The transferred concentrated liquid C is cooled from 60 to 100°C (preferably 70 to 90°C) to 40 to 70°C in the crystallization apparatus, and as a result an adduct crystal precipitates to form a slurry liquid. Next, the slurry liquid containing the adduct crystal dispersed therein is subjected to solid-liquid separation to obtain a mother liquor D and a cake d. The obtained mother liquor D contains unprecipitated bisphenol A and phenol. The cake d contains the adduct crystal as the main component.

The solid-liquid separation can be carried out by a known means such as filtration and centrifugation. For example, solid-liquid separation can be carried out, for example, by using a horizontal belt filter, a rotary vacuum filter, a rotary pressure filter, a batch filtering device, a centrifugal filtering separator, a centrifugal sedimentation separator, or a hybrid centrifuge using any of them (screen ball decanter).

[Step E]

The step E is a step of purifying the cake d obtained in the step D to obtain bisphenol A. The method for purifying the cake d is not particularly limited, and phenol is separated from the cake d obtained in the step D to recover bisphenol A, for example, by a method of removing phenol from a melt formed by heat-melting the cake d, or a method of crystallizing with a hydrocarbon solvent such as toluene.

As the method for removing phenol from the cake d, a method is employed in which the cake d is heat-melted usually at 100 to 160°C, and most of phenol is removed from the resulting melt, for example, by using a distillation apparatus, a thin-film evaporator, or a flush evaporator. To remove a trace amount of phenol remaining in the melt, a method is also employed in which, after the aforementioned operation is carried out, remaining phenol is further removed to purify bisphenol A by steam stripping or the like. This method is described in, for example, Japanese Patent Laid-Open No. 63-132850 and Japanese Patent Laid-Open No. 2-28126.

The high-purity bisphenol A in a melted state obtained as described is sent to a granulation column or a flaker, and processed into solid prills or flakes as a product of bisphenol A. Alternatively, the obtained bisphenol A can be directly transferred in a melted state to the next step without processing into a solid, as with the case of subjecting to production of a polycarbonate resin by the melting method.

[Step F]

The step F is a step of circulating part of the mother liquor D obtained in the step D to supply to the dehydration condensation in the step B.

Part of the mother liquor D obtained through solid-liquid separation is passed through piping connected to the condensation reaction apparatus that carries out the step B to be supplied to the condensation reaction apparatus, and subjected to the step B. All or part of the rest of the mother liquor D obtained through solid-liquid separation is passed through piping connected to an apparatus that carries out the step G or the step H to be supplied to the step G or the step H.

[Step G]

The step G is a step of mixing the crude solution A obtained in the step A and part of the mother liquor D obtained in the step D to obtain a mixed liquid G.

The mixed liquid G may be prepared by mixing the crude solution A and the mother liquor D before transferring to a reaction column that carries out the step H, and, alternatively, the crude solution A and the mother liquor D can be each transferred to a reaction column (apparatus) that carries out the step H to prepare the mixed liquid G in the reaction column.

The step G is a step that is carried out in the case that the mixed liquid G containing the crude solution A and the mother liquor D is treated in the step H. As shown as the pathway P2 in Figure 1, after preliminary mixing of the crude solution A and the mother liquor D, the resulting mixed liquid G can be supplied to an apparatus that carries out degradation of bisphenol A in the step H. Alternatively, as shown as the pathway P1 in Figure 1, the crude solution A and part of the mother liquor D obtained in the step D may be each supplied to an apparatus that carries out degradation of bisphenol A in the step H to carry out the degradation in the step H while the crude solution A and part of the mother liquor D are mixed in the reaction column.

Regarding the mixing ratio between the crude solution A and the mother liquor D, an excessively large amount of the crude solution A results in larger amounts of components that cannot be degraded in the step H, which may promote solidification and clogging of piping to transfer from a reaction tank that carries out the step H. Accordingly, the mass ratio of the crude solution A to the mother liquor D (mass of crude solution A / mass of mother liquor D) is preferably 10 or less, and can be appropriately set to, for example, 5 or less, 2 or less, 1 or less, for example, according to the bisphenol A content of the crude solution A. The lower limit of the mass ratio of the crude solution A to the mother liquor D is not particularly limited, and can be appropriately set to, for example, 0.0001 or more, 0.0001 or more, 0.001 or more, or 0.01 or more, 0.1 or more, for example, according to the bisphenol A content of the crude solution A.

The bisphenol A content of the mixed liquid G is preferably 0.1% by mass or more. Excessively high bisphenol A content causes the precipitation of bisphenol A to clog piping, thus being unpreferable. Excessively low bisphenol A content results in a smaller amount of isopropenylphenol or acetone to be obtained by degradation, thus being unpreferable.

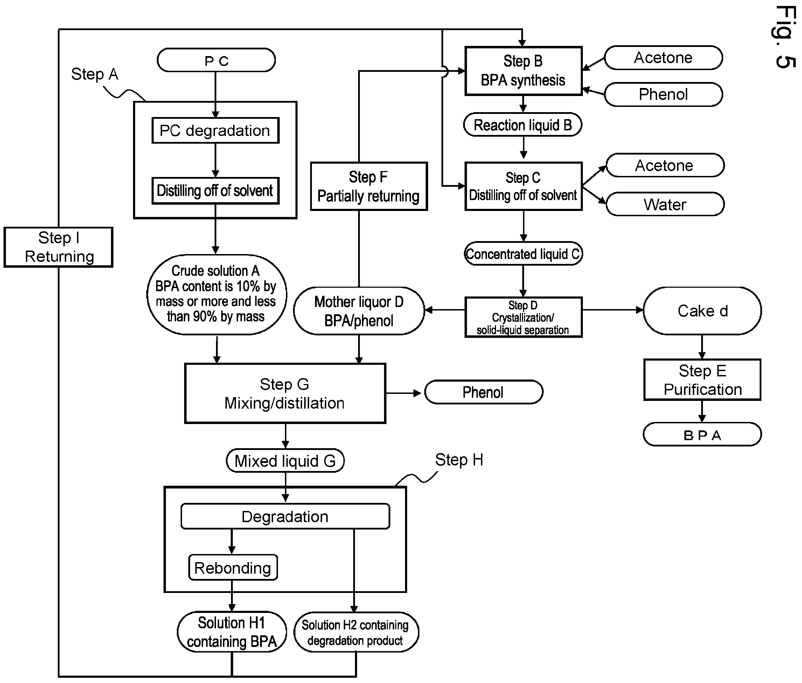

The step G may be a step of mixing the crude solution A obtained in the step A and part of the mother liquor D obtained in the step D and carrying out distillation to obtain a mixed liquid G (see Figure 5). For example, the mixed liquid G can be prepared by transferring the crude solution A and the mother liquor D to a distillation column to mix the crude solution A and the mother liquor D, and further distilling off part of phenol.

Distillation can be carried out at a temperature of 100 to 250°C and a pressure of 1 kPa to 100 kPa.

Part of the mother liquor D may be subjected to treatment such as isomerization, concentration, and crystallization/solid-liquid separation before use in the step H. For example, as illustrated in Figure 1, a step f1 of subjecting reaction byproducts in the mother liquor D obtained in the step D to isomerization treatment and a step f2 of subjecting a solution S3 containing the solution S3a after the isomerization treatment in the step f1 to crystallization and solid-liquid separation may be carried out.

[Step f1]

The step f1 is a step of subjecting reaction byproducts in the mother liquor D obtained in the step D to isomerization treatment. The composition of the mother liquor D obtained in the step D is usually composed of 65 to 85% by mass of phenol, 1 to 20% by mass of bisphenol A, and 1 to 15% by mass of byproducts of bisphenol A such as 2,4-bisphenol A, and the mother liquor D contains much impurities such as an isomer of bisphenol A (2,4-bisphenol A). Through the isomerization treatment, the isomer of bisphenol A is converted into bisphenol A (2,2-bisphenol A). The solution S3a after the isomerization treatment contains about 15 to 20% by mass of bisphenol A and about 5 to 10% by mass of byproducts such as 2,4-bisphenol A. Part of the solution S3a after the isomerization treatment is withdrawn and sent to the step f2 in order to prevent impurities from accumulating. Alternatively, part of the solution S3a after the isomerization treatment may be recirculated to at least one of the step B, the step C, and the step D.

In the isomerization treatment, a cation-exchange resin of sulfonic acid type is usually used as a catalyst, the reaction temperature is about 50 to 100°C, and, in the case of the fixed-bed flow method, which is a continuous washing-away method, the liquid hourly space velocity (LHSV) is about 0.2 to 50/h.

[Step f2]

The step f2 is a step of subjecting a solution S3 containing the solution S3a after the isomerization treatment in the step f1 to crystallization and solid-liquid separation. The solution S3 is the solution S3a (pathways P3, P4 in Figure 1), or a solution S3b containing the solution S3a and the crude solution A (pathway P5 in Figure 1). The liquid after the isomerization treatment in the step f1 may be directly used as the solution S3a, and the solution S3a may be a concentrated liquid obtained by removing part of phenol from the liquid after the isomerization treatment in the step f1. A liquid containing the solution S3a and the crude solution A may be directly used as the solution S3b, and the solution S3b may be a concentrated liquid obtained by removing part of phenol from the liquid containing the solution S3a and the crude solution A. The concentration of bisphenol A in the liquid after concentration is about 20 to 50% by mass. The concentration operation can be carried out with a distillation column or the like, and is performed at a pressure of about 5.3 to 40 kPa and a temperature of about 70 to 140°C. Evaporated phenol may be reused as a washing liquid for washing a cake of an adduct crystal resulting from solid-liquid separation.

The solution S3a is cooled to crystalize an adduct crystal of bisphenol A and phenol, giving a slurry liquid. The solution S3a may be supplied to a crystallization apparatus after being cooled to a temperature near the freezing point by using a heat exchanger with warm water as a cooling medium in advance of supplying to the crystallization apparatus. Next, the slurry liquid with the adduct crystal resulting from the crystallization is subjected to solid-liquid separation to obtain a cake of the adduct crystal and a mother liquor S2a (pathways P3, P4 in Figure 1). The solid-liquid separation can be carried out by a known method as in the step D.

In the case of the solution S3b, similarly, the solution S3b is cooled to obtain a slurry liquid, and the slurry liquid is subjected to solid-liquid separation to obtain a cake of the adduct crystal and a mother liquor S2b (pathway P5 in Figure 1).

The obtained mother liquor S2a and mother liquor S2b are subjected to the step H. The cake of the adduct crystal is melted and then recirculated to the step C and/or the step D.

The step f1 and the step f2 are pretreatment steps carried out in the case that the mother liquor S2a and the crude solution A are treated in the step H or that the mother liquor S2b is treated in the step H. As shown as the pathway P4 in Figure 1, the mother liquor S2a obtained by subjecting the mother liquor D to the step f1 and the step f2 and the crude solution A can be mixed in advance and then supplied to an apparatus that carries out degradation of bisphenol A in the step H. Alternatively, as shown as the pathway P3 in Figure 1, the mother liquor S2a obtained by subjecting the mother liquor D to the step f1 and the step f2 and the crude solution A may be each supplied to an apparatus that carries out degradation of bisphenol A in the step H and subjected to degradation in the step H while the crude solution A and the mother liquor S2a are mixed in the reaction column. The mother liquor S2a and the crude solution A may be separately treated in the step H without mixing. Alternatively, as shown as the pathway P5 in Figure 1, the S3a after the isomerization treatment of the mother liquor D in the step f1 and the crude solution A are mixed, the resultant is subjected to the step f2, and the resulting mother liquor S2b can be then supplied to an apparatus that carries out degradation of bisphenol A in the step H.

[Step H]

The step H is any of the following (I) to (III).

(I) A step of obtaining a solution H1 or a solution H2 from the crude solution A and part of the mother liquor D

The solution H1 in (I) is a solution containing bisphenol A obtained by degrading bisphenol A contained in the crude solution A and the mother liquor D into phenol and isopropenylphenol under conditions that allow degradation of bisphenol A and then rebonding phenol and isopropenylphenol. The solution H2 in (I) is a solution obtained by degrading bisphenol A contained in the crude solution A and the mother liquor D into phenol and acetone under conditions that allow degradation of bisphenol A.

(II) A step of subjecting part of the mother liquor D to isomerization treatment and crystallization/solid-liquid separation treatment and obtaining a solution H1 or a solution H2 from a mother liquor S2a obtained and the crude solution A

The solution H1 in (II) is a solution containing bisphenol A obtained by degrading bisphenol A contained in the mother liquor S2a and the crude solution A into phenol and isopropenylphenol under conditions that allow degradation of bisphenol A and then rebonding phenol and isopropenylphenol. The solution H2 in (II) is a solution obtained by degrading bisphenol A contained in the mother liquor S2a and the crude solution A into phenol and acetone under conditions that allow degradation of bisphenol A.

(III) A step of subjecting a solution S3b containing a solution S3a after isomerization treatment of the mother liquor D and the crude solution A to crystallization/solid-liquid separation treatment and obtaining a solution H1 or a solution H2 from a mother liquor S2b obtained

The solution H1 in (III) is a solution containing bisphenol A obtained by degrading bisphenol A contained in the mother liquor S2b into phenol and isopropenylphenol under conditions that allow degradation of bisphenol A and then rebonding phenol and isopropenylphenol. The solution H2 in (III) is a solution obtained by degrading bisphenol A contained in the mother liquor S2b into phenol and acetone under conditions that allow degradation of bisphenol A.

Hereinafter, the step H will be described in more detail for the case that a solution H1 or a solution H2 is obtained from the mixed liquid G containing the crude solution A and the mother liquor D (Figure 2) as an example.

The step H is a step of obtaining a solution H1 containing bisphenol A, wherein the solution H1 is obtained by treating the mixed liquid G under conditions that allow degradation of bisphenol A and rebonding, or a solution H2 containing a degradation product, wherein the solution H2 is obtained by treating the mixed liquid G under conditions that allow degradation of bisphenol A.

In the step H, usually, distillation is carried out after or during the mixed liquid G is treated under conditions that allow degradation of bisphenol A, a fraction h containing a degradation product is recovered, and the residue is removed. The amount of the residue to be removed is appropriately adjusted according to the amount of the mixed liquid G to be subjected to the step H.

In the step H, the degradation rate of bisphenol A in the mixed liquid G after treating the mixed liquid G under conditions that allow degradation of bisphenol A is preferably 20 mol% or more, and more preferably 30 mol% or more. As described above, the degradation product is evaporated by distillation in the step H. At that time, components that have not been degraded remain in the residue without evaporation. If the degradation rate is too low, more components remain in the residue without evaporation. The components are to be dealt with as wastes, thus being unpreferable. The degradation rate of bisphenol A in the mixed liquid G can be determined as the amount of the degradation product contained in the fraction h relative to the amount of bisphenol A in the mixed liquid G (number of moles of degradation product / number of moles of bisphenol A in mixed liquid G $\times$ 100 (%)). In the step H1 described later, for example, the degradation rate can be determined as the amount of isopropenylphenol contained in the fraction h1 relative to the amount of bisphenol A in the mixed liquid G.

The conditions that allow degradation of bisphenol A are any selected from the group consisting of alkaline conditions that allow alkaline degradation of bisphenol A in the presence of an alkaline catalyst, acidic conditions that allow acidic degradation of bisphenol A in the presence of an acid catalyst, and supercritical water conditions that allow degradation of bisphenol A in supercritical water.

Examples of the basic catalyst applicable in subjecting bisphenol A to alkaline degradation under alkaline conditions include hydroxides, oxides, carbonates, and various phenoxides of alkali metals such as sodium and potassium, and hydroxides, oxides, carbonates, and various phenoxides of alkaline earth metals such as calcium and magnesium. Among these, sodium hydroxide or potassium hydroxide is preferable.

Examples of the acid catalyst applicable in subjecting bisphenol A to acidic degradation under acidic conditions include alkylsulfonic acids such as methanesulfonic acid, aromatic sulfonic acids such as toluenesulfonic acid, phenolsulfonic acid, and cresolsulfonic acid, inorganic acids such as sulfuric acid, hydrogen chloride, phosphoric acid, nitric acid, and phosphinic acid, titanium oxide, zirconium oxide, and acidic alumina. Among these, sulfonic acids are preferable, aromatic sulfonic acids are more preferable, and toluenesulfonic acid is further preferable.

The "supercritical water" for use in supercritical water degradation of bisphenol A refers to water in a state over the critical point of water (374 °C, 22 MPa) (374 °C, 218 atm).

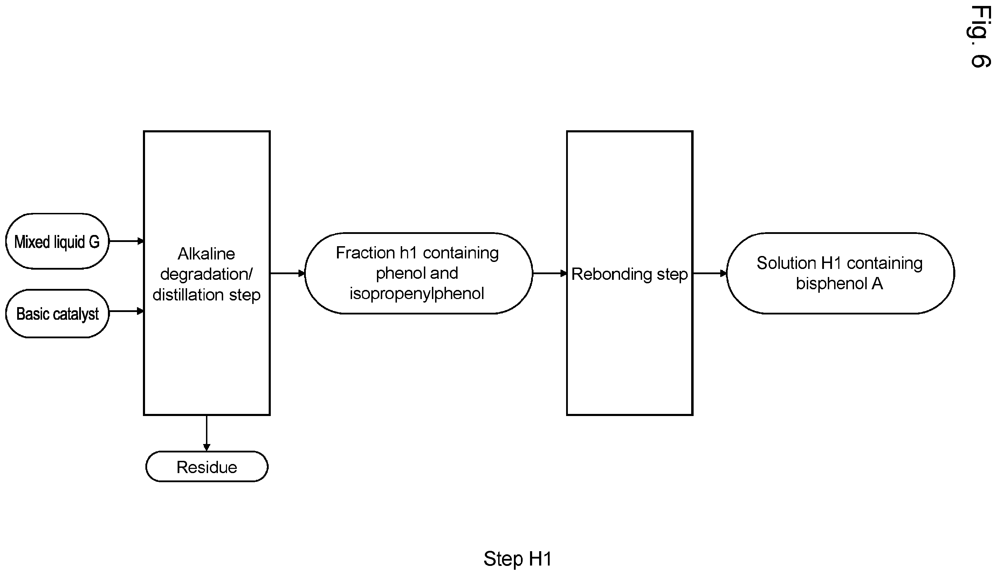

Hereinafter, treatments of the mixed liquid G under alkaline conditions, under acidic conditions, and under supercritical water conditions will be specifically described with reference to Figure 6 to Figure 9.

(i) Alkaline degradation

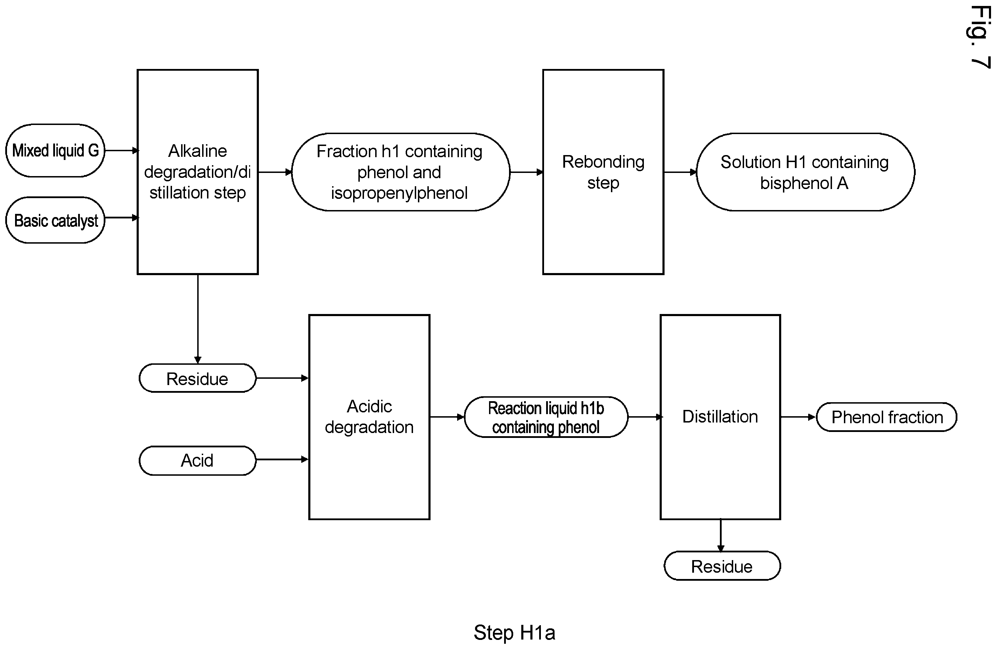

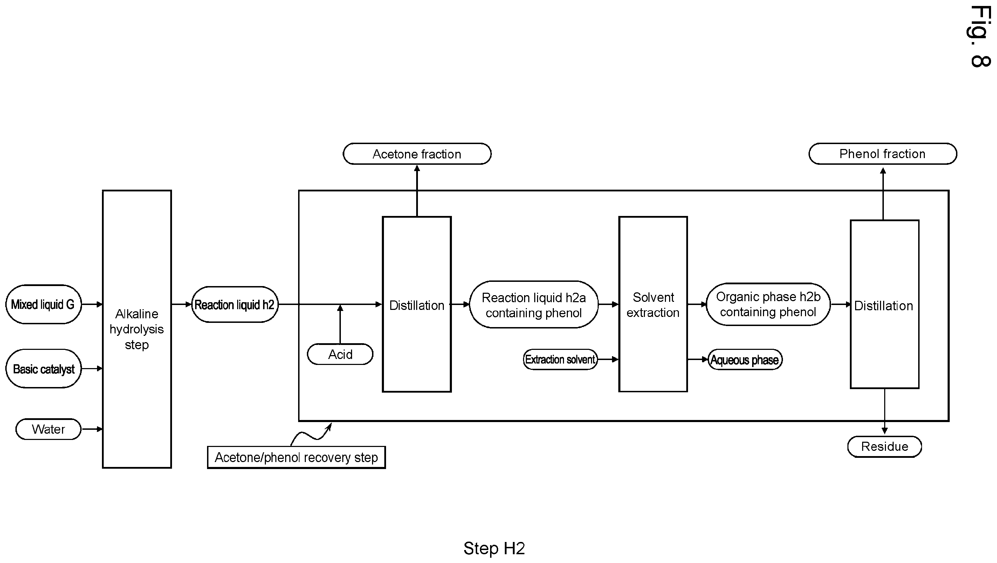

The step H1 illustrated in Figure 6, the step H1a illustrated in Figure 7, and the step H2 illustrated in Figure 8 each include a step of subjecting bisphenol A in the mixed liquid G to alkaline degradation in the presence of a basic catalyst.

[Step H1]

The step H1 illustrated in Figure 6 is an example of the step of obtaining a solution H1 containing bisphenol A, wherein the solution H1 is obtained by treating the mixed liquid G under conditions that allow degradation of bisphenol A and then rebonding. The step H1 includes: an alkaline degradation/distillation step of carrying out distillation while the mixed liquid G is treated under alkaline conditions that allow degradation of bisphenol A to obtain a fraction h1 containing phenol and isopropenylphenol as a degradation product; and a rebonding step of rebonding phenol and isopropenylphenol contained in the fraction h1 to generate bisphenol A. Having high reaction activity, isopropenylphenol, which is generated through degradation of bisphenol A, disadvantageously forms condensates other than bisphenol A if it is not rebonded quickly. Distillation while the mixed liquid G is treated under alkaline conditions that allow degradation of bisphenol A to degrade bisphenol A as in the step H1 allows generated isopropenylphenol to quickly evaporate and to be supplied to the rebonding step, thus successfully suppressing side reactions of isopropenylphenol.

(Alkaline degradation/distillation step)

The alkaline degradation/distillation step can be carried out, for example, with a reaction-distillation apparatus including a reaction tank in the bottom and a distillation column in the top. The mixed liquid G and a basic catalyst are transferred to the reaction tank in the bottom, and heated to degrade bisphenol A contained in the mixed liquid G into phenol and isopropenylphenol. At that time, incomplete degradation products of the polycarbonate resin (multimers, such as dimers and trimers, of bisphenol A), an isomer of bisphenol A (2,4-bisphenol), and others are also degraded into phenol and isopropenylphenol. Impurities such as chroman compounds undergo heavy component formation reaction and are converted into high-boiling-point substances (compounds having higher boiling point than bisphenol A). To efficiently degrade bisphenol A and the like into phenol and isopropenylphenol, the moisture content of the mixed liquid G is usually adjusted to 0.01% by mass or less.

The temperature in carrying out the alkaline degradation/distillation (i.e., the temperature in the reaction tank) is preferably 180°C or more, and more preferably 200°C or more. The temperature in carrying out the alkaline degradation/distillation is preferably 350°C or less, and more preferably 300°C or less.

The alkaline degradation/distillation is usually carried out at an intracolumn pressure of 0.6 kPa to normal pressure, and can be preferably carried out under a pressure condition of 13 to 20 kPa.

Phenol and isopropenylphenol generated through degradation evaporate, are withdrawn from the column top of the reaction column to be recovered as a fraction h1, and transferred to the reaction tank that carries out the rebonding step. At that time, phenol may be supplied together with the fraction h1 to the reaction tank that carries out the rebonding step to achieve a specific phenol-isopropenylphenol ratio. In the reaction liquid remaining in the reaction tank without evaporation, high-boiling-point substances and substances that cause coloring of bisphenol A are concentrated. The residue is removed by withdrawing the reaction liquid from the column bottom of the reaction column.

The fraction h1 preferably contains 1.0% by mass or more of isopropenylphenol. If the isopropenylphenol content of the fraction h1 is less than 1.0% by mass, less bisphenol A is recovered after rebonding. The fraction h1 preferably contains 50% by mass or less of isopropenylphenol, and more preferably contains 30% by mass or less of isopropenylphenol. This is because excessively high isopropenylphenol content causes the formation of condensates other than bisphenol A.

The fraction h1 preferably contains isopropenylphenol and phenol, and the phenol content of the fraction h1 is preferably 99% by mass or less. The phenol content is preferably 50% by mass or more, and preferably 70% by mass or more.

(Rebonding step)

In the rebonding step, phenol and isopropenylphenol are rebonded by bringing them into contact with an acid catalyst to generate bisphenol A. Thereby, phenol and isopropenylphenol condense to generate bisphenol A, giving a solution H1 containing bisphenol A. The acid catalyst for rebonding is preferably a highly acidic cation-exchange resin of sulfonic acid type, and rebonding can be carried out, for example, with a reaction apparatus packed with a highly acidic cation-exchange resin of sulfonic acid type.

The reaction temperature is usually 45 to 130°C, and preferably 50 to 100°C. The time for contact with the acid catalyst is usually 5 to 200 minutes, and preferably 15 to 120 minutes.

The obtained solution H1 preferably contains 1.0% by mass or more of bisphenol A. If the bisphenol A content of the solution H1 is less than 1.0% by mass, components other than bisphenol A are much generated to increase the amount of impurities mixed in bisphenol A, and thus the bisphenol A obtained in the step E tends to have deteriorated quality. The solution H1 preferably contains 50% by mass or less of bisphenol A, and more preferably 40% by mass or less of bisphenol A. This is because excessively high bisphenol A content causes the precipitation of bisphenol A generated, promoting clogging of piping to transfer the solution H1 from the reaction tank where rebonding was carried out to the reaction apparatus that carries out the step B and/or the step C.

The step H1 may include a step of degrading a residue (a reaction liquid remaining in the reaction tank without evaporation) with an acid catalyst. Thereby, substances that were not degraded in the alkaline degradation/distillation step are degraded and phenol is generated. For example, as in the step H1a illustrated in Figure 7, the residue is degraded in the presence of an acid catalyst at 150 to 300°C, and phenol can be recovered through distillation of the resulting reaction liquid h1b containing phenol (temperature: 150 to 300°C, pressure: 0.1 kPa to 10 kPa), and this phenol can also be returned to the step B and/or the step C. Examples of the acid catalyst at that time include aromatic sulfonic acids such as p-toluenesulfonic acid. The residue is basic, and hence the amount of the acid catalyst to be mixed is controlled so that the residue can be degraded under acidic conditions.

[Step H2]

The step H2 illustrated in Figure 8 is an example of the step of obtaining a solution H2 containing a degradation product, wherein the solution H2 is obtained by treating the mixed liquid G under alkaline conditions that allow degradation of bisphenol A. The step H2 includes: an alkaline hydrolysis step of treating the mixed liquid G under conditions that allow hydrolysis of bisphenol A to obtain a reaction liquid h2 containing acetone and phenol; and an acetone/phenol recovery step of recovering acetone and/or phenol in the reaction liquid h2 obtained in the alkaline hydrolysis step and removing a residue. The step H2 in this manner is preferable because water, the basic catalyst, acetone, phenol, and the residue contained in the reaction liquid h2 can be efficiently separated.

(Alkaline hydrolysis step)

In the alkaline hydrolysis step, for example, the mixed liquid G, a basic catalyst, and water are transferred to a reaction apparatus, and heated to degrade bisphenol A contained in the mixed liquid G into acetone and phenol. At that time, incomplete degradation products of the polycarbonate resin (multimers, such as dimers and trimers, of bisphenol A), an isomer of bisphenol A (2,4-bisphenol), and others are also degraded into phenol and acetone. Impurities such as chroman compounds undergo heavy component formation reaction and are converted into high-boiling-point substances (compounds having higher boiling point than bisphenol A). As a result, a reaction liquid h2 containing acetone, phenol, impurities, and others is obtained.

Too much water supplied to the reaction apparatus together with the mixed liquid G tends to lower the degradation efficiency, and too little water tends to result in the increase in the amount of components that cannot be degraded and significantly lower the alkaline hydrolysis rate. The mass ratio of water to the mixed liquid G (mass of water / mass of mixed liquid G) is preferably 1 or more, and more preferably 1.5 or more. The mass ratio is preferably 300 or less, and more preferably 100 or less.

The temperature in carrying out hydrolysis is preferably 180°C or more, and more preferably 200°C or more. The temperature in carrying out hydrolysis is preferably 350°C or less, and more preferably 300°C or less. The pressure in hydrolysis is usually the vapor pressure at the temperature.

The reaction liquid h2 preferably contains 0.1% by mass or more of acetone. If the acetone content of the reaction liquid h2 is less than 1.0% by mass, it is difficult to properly recover acetone. The reaction liquid h2 preferably contains 30% by mass or less of acetone, and more preferably 20% by mass or less of acetone. This is because excessively high acetone content causes the self-condensation of acetone to lower the recovery rate for acetone.

(Acetone/phenol recovery step)

In the acetone/phenol recovery step, the reaction liquid h2 obtained in the alkaline hydrolysis step is first neutralized, and subjected to distillation to recover acetone. In a reaction liquid h2a remaining without evaporation, phenol, impurities, and others are concentrated. Next, phenol in the reaction liquid h2a is subjected to solvent extraction to obtain an organic phase h2b containing phenol, and the organic phase h2b is then subjected to distillation to recover phenol. Impurities can be removed by removing a residue remaining after distillation of phenol. The recovered acetone and phenol are used as the solution H2 to be subjected to the step B and/or the step C.

Specifically, the reaction liquid h2 is neutralized and transferred to an acetone recovery distillation column, and acetone is distilled under temperature and pressure conditions of 30 to 200°C and 0.1 to 100 kPa and withdrawn from the column top. The reaction liquid h2a in the distillation column (column bottom liquid) is transferred to a solvent extraction apparatus.

The reaction liquid h2a is treated with a water-immiscible organic solvent in the solvent extraction apparatus to obtain an organic phase h2b containing phenol and an aqueous phase, which are separately withdrawn. Examples of the water-immiscible organic solvent include ethers such as tert-butyl methyl ether, tert-amyl ethyl ether, and diisopropyl ether; ketones such as methyl ethyl ketone and methyl isobutyl ketone; and acetates such as propyl acetate, butyl acetate, pentyl acetate, and hexyl acetate.

The withdrawn organic phase h2b containing phenol is transferred to a phenol recovery distillation column, and phenol is distilled under temperature and pressure conditions of 100 to 300°C and 0.1 to 10 kPa and withdrawn from the column top. A residue remaining in the phenol recovery distillation column (column bottom liquid) is withdrawn from the column bottom.

In the acetone/phenol recovery step, acetone and phenol may be each recovered in such a manner that acetone is distilled from the reaction liquid h2 and withdrawn, the residual reaction liquid is neutralized, and phenol is distilled.

(ii) Acidic degradation

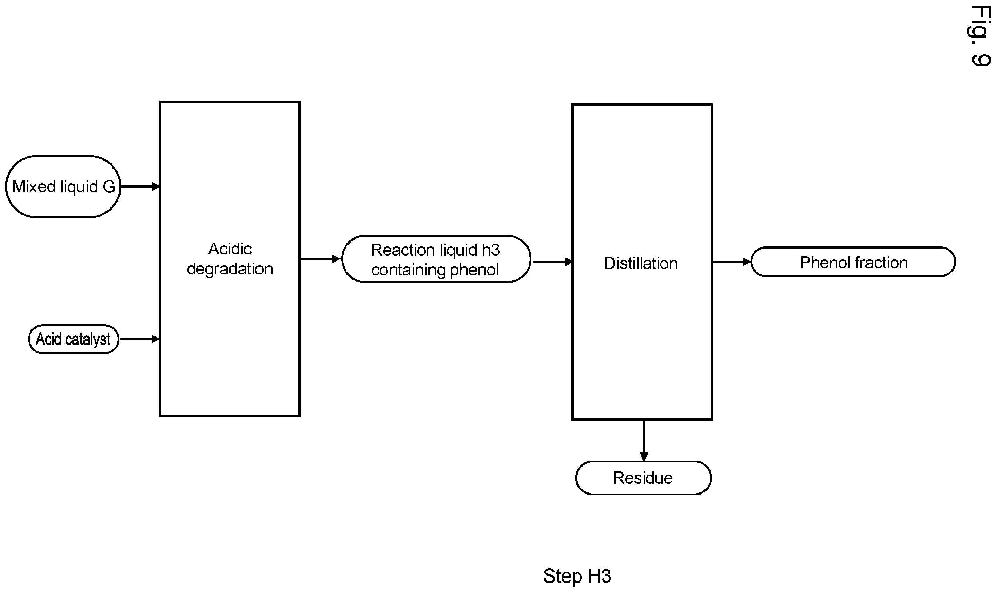

The step H3 illustrated in Figure 9 is a step of treating the mixed liquid G under acidic conditions that allow degradation of bisphenol A. In the step H3, a solution H2 containing a degradation product is obtained by treating the mixed liquid G under conditions that allow degradation of bisphenol A.

[Step H3]

The step H3 illustrated in Figure 9 includes: an acidic degradation step of treating the mixed liquid G under acidic conditions that allow degradation of bisphenol A to obtain a reaction liquid h3 containing phenol; and a phenol recovery step of subjecting the reaction liquid h3 obtained in the acidic degradation step to distillation to recover a fraction containing phenol and removing a residue. The step H3 in this manner can improve the recovery rate for phenol, thus being preferable.

The temperature in carrying out the acidic degradation is preferably 100°C or more, and more preferably 150°C or more. The temperature in carrying out the acidic degradation is preferably 300°C or less, and more preferably 250°C or less. The pressure in the acidic degradation can be set to 0.1 to 10 kPa. The reaction liquid h3 obtained in the acidic degradation step can be subjected to distillation at a temperature of 150 to 300°C and a pressure of 0.1 to 10 kPa.

(iii) Supercritical water degradation

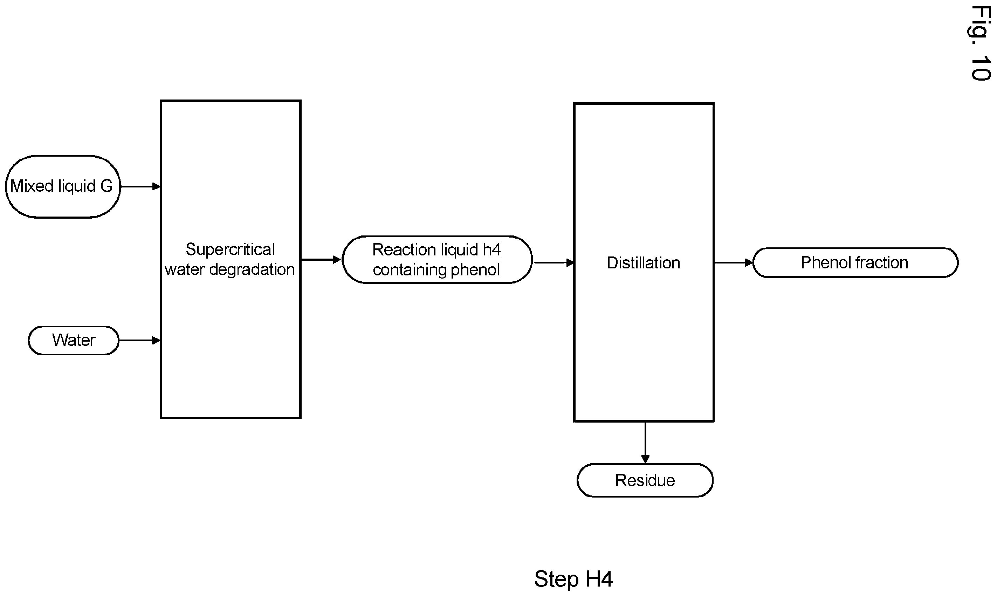

The step H4 illustrated in Figure 10 is a step of treating the mixed liquid G under supercritical water conditions that allow degradation of bisphenol A. Bisphenol A can be degraded into phenol and acetone by using supercritical water.

[Step H4]

The step H4 illustrated in Figure 10 includes: a supercritical water degradation step of treating the mixed liquid G under supercritical water conditions that allow degradation of bisphenol A to obtain a reaction liquid h4 containing phenol; and a phenol recovery step of subjecting the reaction liquid h4 obtained in the supercritical water degradation step to distillation to recover a fraction containing phenol and removing a residue. The step H4 in this manner allows degradation without use of a catalyst, thus being preferable.

In the supercritical water degradation step, the mixed liquid G and water are added into a reaction apparatus, water is converted into the supercritical state by raising the temperature and pressure over the critical point of water, and the resulting supercritical water can degrade bisphenol A therein. Specifically, the temperature can be set to 300 to 700°C, preferably to 350 to 500°C.

The reaction liquid h4 obtained in the supercritical water degradation step can be subjected to distillation at a temperature of 150 to 300°C and a pressure of 0.1 to 10 kPa.

[Step I]

The step I is a step of supplying the solution H1 or the solution H2 obtained in the step H to the step B and/or the step C.

For example, via piping provided to connect the reaction apparatus that carries out the rebonding reaction, the acetone recovery distillation column, or the phenol recovery distillation column to the condensation reaction apparatus that carries out the step B, the solution H1 withdrawn from the reaction apparatus that carries out the rebonding reaction, acetone withdrawn from the acetone recovery distillation column, or phenol withdrawn from the phenol recovery distillation column can be subjected to the step B.

Alternatively, via piping provided to connect the reaction apparatus that carries out the rebonding reaction, the acetone recovery distillation column, or the phenol recovery distillation column to the distillation column that carries out the step C, the solution H1 withdrawn from the reaction apparatus that carries out the rebonding reaction, acetone withdrawn from the acetone recovery distillation column, or phenol withdrawn from the phenol recovery distillation column can be subjected to the step C.

The crude solution A and the mother liquor D may be separately supplied to and treated in the apparatus that carries out the step H, or separately treated in the step H without mixing together. The (II) is the same as the method (I) for obtaining the solution H1 or the solution H2, except that the mother liquor S2a and the crude solution A are used. The (III) is the same as the method (I) for obtaining the solution H1 or the solution H2, except that the mother liquor S2b is used.

<Second method for producing bisphenol A>

**Disclosed herein is** a method for producing bisphenol A, including the following step B to step F, step H, and step I (hereinafter, sometimes referred to as the "second method for producing bisphenol A").

Step B: a step of subjecting acetone and phenol to dehydration condensation in the presence of an acid catalyst to obtain a reaction liquid B containing bisphenol A
Step C: a step of distilling off unreacted acetone and water from the reaction liquid B obtained in the step B to obtain a concentrated liquid C
Step D: a step of subjecting the concentrated liquid C obtained in the step C to crystallization to obtain a slurry liquid, and subjecting the slurry liquid to solid-liquid separation to obtain a mother liquor D and a cake d
Step E: a step of purifying the cake d obtained in the step D to obtain bisphenol A
Step F: a step of circulating part of the mother liquor D obtained in the step D to supply to the step B
Step H: any step of the following (I) to (III)

(I) a step of obtaining a solution H1 or a solution H2 from the crude solution A and part of the mother liquor D

The crude solution A in (I) is obtained by degrading a polycarbonate resin in a solvent to obtain a reaction liquid a1 containing bisphenol A and then distilling off the solvent from the obtained reaction liquid a1, and the bisphenol A content of the crude solution A is less than 90% by mass. The solution H1 in (I) is a solution containing bisphenol A obtained by degrading bisphenol A contained in the crude solution A and the mother liquor D into phenol and isopropenylphenol under conditions that allow degradation of bisphenol A and then rebonding phenol and isopropenylphenol. The solution H2 in (I) is a solution obtained by degrading bisphenol A contained in the crude solution A and the mother liquor D into phenol and acetone under conditions that allow degradation of bisphenol A.

(II) a step of subjecting part of the mother liquor D to isomerization treatment and crystallization/solid-liquid separation treatment and obtaining a solution H1 or a solution H2 from a mother liquor S2a obtained and the crude solution A

The crude solution A in (II) is obtained by degrading a polycarbonate resin in a solvent to obtain a reaction liquid a1 containing bisphenol A and then distilling off the solvent from the obtained reaction liquid a1, and the bisphenol A content of the crude solution A is less than 90% by mass. The solution H1 in (II) is a solution containing bisphenol A obtained by degrading bisphenol A contained in the mother liquor S2a and the crude solution A into phenol and isopropenylphenol under conditions that allow degradation of bisphenol A and then rebonding phenol and isopropenylphenol. The solution H2 in (II) is a solution obtained by degrading bisphenol A contained in the mother liquor S2a and the crude solution A into phenol and acetone under conditions that allow degradation of bisphenol A.

(III) a step of subjecting a solution S3b containing a solution S3a after isomerization of the mother liquor D and the crude solution A to crystallization/solid-liquid separation treatment and obtaining a solution H1 or a solution H2 from a mother liquor S2b obtained

The crude solution A in (III) is obtained by degrading a polycarbonate resin in a solvent to obtain a reaction liquid a1 containing bisphenol A and then distilling off the solvent from the obtained reaction liquid a1, and the bisphenol A content of the crude solution A is less than 90% by mass. The solution H1 in (III) is a solution containing bisphenol A obtained by degrading bisphenol A contained in the mother liquor S2b into phenol and isopropenylphenol under conditions that allow degradation of bisphenol A and then rebonding phenol and isopropenylphenol. The solution H2 in (III) is a solution obtained by degrading bisphenol A contained in the mother liquor S2b into phenol and acetone under conditions that allow degradation of bisphenol A.

Step I: a step of supplying the solution H1 or the solution H2 obtained in the step H to the step B and/or the step C

The second method for producing bisphenol A is the same as the method of the present invention for producing bisphenol A, except that production of the crude solution A may be carried out in a single facility that is not integrated with and independent of the facility that carries out the step B to the step F, the step H, and the step I. In the second method for producing bisphenol A, the crude solution A can be produced in the same manner as in the step A of the method of the present invention for producing bisphenol A. The step B to the step F, the step H, and the step I of the second method for producing bisphenol A are the same as the step B to the step F, the step H, and the step I of the method of the present invention for producing bisphenol A.

The crude solution A and part of the mother liquor D can be mixed and subjected to the step H. At that time, the crude solution A and part of the mother liquor D can be mixed in advance and the resulting mixed liquid G can be supplied to the apparatus that carries out degradation of bisphenol A. The mixed liquid G may be supplied to the step H after concentration as appropriate. Alternatively, the crude solution A and part of the mother liquor D may be each supplied to the apparatus that carries out degradation of bisphenol A in the step H and subjected to degradation reaction while the mixed liquid G is prepared in the apparatus. The mixing ratio or the like between the crude solution A and part of the mother liquor D is the same as that in the step G of the method of the present invention for producing bisphenol A. The crude solution A and the mother liquor D may be separately treated in the step H without mixing.

Part of the mother liquor D may be subjected to treatment such as isomerization, concentration, and crystallization/solid-liquid separation before use in the step H. For example, a step f1 of subjecting reaction byproducts in the mother liquor D obtained in the step D to isomerization treatment and a step f2 of subjecting a solution S3 containing the solution S3a after the isomerization treatment in the step f1 to crystallization and solid-liquid separation may be carried out. Through the step f1 and the step f2, a mother liquor S2a or a mother liquor S2b can be obtained. The step f1 and the step f2 are the same as those of the method of the present invention for producing bisphenol A. In the (II) and the (III), the step G or the step H can be carried out by using the mother liquor S2a or the mother liquor S2b in the same manner as in the (I).

<Application of bisphenol A>

The bisphenol A obtained by the method of the present invention for producing bisphenol A or the second method for producing bisphenol A (hereinafter, sometimes referred to as the "bisphenol A of the present invention") can be used as a constituent component of various thermoplastic resins such as a polyether resin, a polyester resin, a polyarylate resin, a polycarbonate resin, a polyurethane resin, or an acrylic resin, various thermosetting resins such as an epoxy resin, an unsaturated polyester resin, a phenol resin, a polybenzoxazine resin, or a cyanate resin, which are used in various applications such as an optical material, a recording material, an insulating material, a transparent material, an electronic material, an adhesive material, or a heat resistant material, or the like, a curing agent, an additive, a precursor thereof, or the like. In addition, the bisphenol A is also useful as an additive such as a color developer for a heat sensitive recording material or the like, a fading inhibitor, a microbicidal agent, or an antimicrobial or antifungal agent.

The bisphenol A is preferably used as a raw material (monomer) for a thermoplastic resin or a thermosetting resin among these because it can impart a good mechanical physical property, and more preferably used as a raw material for a polycarbonate resin or an epoxy resin among such resins. In addition, the bisphenol A is also preferably used as a color developer, and is more preferably used particularly in combination with a leuco dye or a discoloration temperature adjusting agent.

<Method for producing polycarbonate resin>

The present invention relates to a method for producing a polycarbonate resin including producing a polycarbonate resin by using bisphenol A obtained by the method of the present invention for producing bisphenol A or the second method for producing bisphenol A (hereinafter, sometimes referred to as the "method of the present invention for producing a polycarbonate resin").

The polycarbonate resin obtained by the method of the present invention for producing a polycarbonate resin can be produced, for example, by a method involving subjecting the bisphenol A of the present invention and a diester carbonate such as diphenyl carbonate to an ester exchange reaction in the presence of an alkali metal compound and/or an alkaline earth metal compound. The ester exchange reaction can be carried out by appropriately selecting a known method. An example with use of the bisphenol A of the present invention and diphenyl carbonate as raw materials will be described below.

In the method for producing a polycarbonate resin, it is preferable to use an excess amount of diphenyl carbonate based on that of the bisphenol A of the present invention. The amount of diphenyl carbonate used based on that of the bisphenol A is preferably large from the viewpoint of the produced polycarbonate resin having few terminal hydroxyl groups and being excellent in thermal stability of the polymer, and is preferably small from the viewpoint of the ester exchange reaction rate being fast and it being easy to produce a polycarbonate resin having a desired molecular weight. Because of these, the amount of diphenyl carbonate used per mol of the bisphenol is usually 1.001 mol or more, preferably 1.002 mol or more, and usually 1.3 mol or less, preferably 1.2 mol or less.

As a method for supplying a raw material, the bisphenol A of the present invention and diphenyl carbonate can also be supplied in a solid state, and it is preferable to melt one or both thereof and supply the same in a liquid state.

When producing a polycarbonate resin by an ester exchange reaction between diphenyl carbonate and bisphenol A, an ester exchange catalyst is usually used. In the method for producing a polycarbonate resin, as this ester exchange catalyst, an alkali metal compound and/or an alkaline earth metal compound is preferably used. One of these may be used, or two or more thereof may be used in any combination and at any ratio. Practically, it is desirable to use an alkali metal compound.

The amount of the catalyst used per mol of bisphenol A or diphenyl carbonate is usually 0.05 μmol or more, preferably 0.08 μmol or more, and further preferably 0.10 μmol or more, in addition, the amount thereof is usually 100 μmol or less, preferably 50 μmol or less, and further preferably 20 μmol or less. When the amount of the catalyst used is within such a range, it is easy to obtain a polymerization activity required for producing a polycarbonate resin having a desired molecular weight, and it is easy to obtain a polycarbonate resin having an excellent polymer color hue, a low level of excessive polymer branching, and excellent fluidity during the molding.

In order to produce a polycarbonate resin by the above method, it is preferable to continuously supply both of the above raw materials to a raw material mixing tank and continuously supply the resulting mixture and an ester exchange catalyst to a polymerization tank.

In the production of a polycarbonate resin by the ester exchange method, both of the raw materials supplied to the raw material mixing tank are usually stirred uniformly and then supplied to the polymerization tank to which a catalyst is added, to produce a polymer.

Examples

Hereinafter, the present invention will be described more specifically with reference to Examples and Comparative Examples, but the present invention is not limited by the following Examples as long as they do not depart from the scope of the present invention.

[Raw material and reagents]

As the polycarbonate resin, the polycarbonate resin "NOVAREX (registered trademark) M7027BF" manufactured by Mitsubishi Engineering-Plastics Corporation was used.

As phenol, toluene, sodium hydroxide, potassium hydroxide, hydrochloric acid, acetonitrile, and cesium carbonate, reagents of FUJIFILM Wako Pure Chemical Corporation were used.

As diphenyl carbonate, a product of Mitsubishi Chemical Corporation was used.

[Preparation of cation-exchange resin]

Cation-exchange resin A: according to Reference Example 1 in the patent literature Japanese Patent Laid-Open No. 2012-201619, DIAION (registered trademark) SK104 completely substituted with phenol was obtained.

Cation-exchange resin B: according to Example 1 in the patent literature WO 2012-108385, 2-(2-mercaptoethyl) pyridine-modified highly acidic cation-exchange resin was obtained.

[Analysis]

Checking of the generation and purity of bisphenol A, and quantification of components inferred to be an undegraded portion of the polycarbonate resin and a stabilizer (regarded as components other than phenol and bisphenol A) were carried out by high performance liquid chromatography under the following procedure and conditions.

- Apparatus: LC-2010A manufactured by Shimadzu Corporation, Waters Corporation 5 μm 150 mm × 4.6 mm ID
- Method: Low pressure gradient method
- Analysis temperature: 40°C
- Eluent composition:

    Liquid A acetonitrile
    Liquid B a solution of 85% phosphoric acid:water = 1 mL:999 mL
    At minute 0 of the analysis time, the eluent composition was liquid A:liquid B = 35:65 (volume ratio, the same applies hereinafter); at minute 0 to minute 5 of the analysis time, the eluent composition was liquid A:liquid B = 35:65; and then at minute 5 to minute 40 of the analysis time, the eluent composition was gradually changed to liquid A:liquid B = 90:10.

- Flow rate: 0.85 mL/min
- Detection wavelength: 280 nm

Analysis of dimethyl carbonate was carried out by gas chromatography under the following procedure and conditions.

- Apparatus: GC-2014 manufactured by Shimadzu Corporation Agilent DB-1 0.530 mm $\times$ 30 m 1.5 $\mu$m
- Detection method: FID
- Vaporization chamber temperature: 230°C
- Detector temperature: 300°C
- At minute 0 to minute 5 of the analysis time, the column temperature was kept at 50°C; at minute 5 to minute 30 of the analysis time, the column temperature was gradually raised to 280°C; and at minute 30 to minute 40 of the analysis time, the column temperature was maintained at 280°C.
- Quantification method: Internal standard method using biphenyl as the internal standard

[Viscosity average molecular weight (Mv)]

The viscosity average molecular weight (Mv) was calculated by dissolving a polycarbonate resin in methylene chloride (concentration of 6.0 g/L), measuring the specific viscosity ($\eta$sp) at 20°C by using an Ubbelohde viscosity tube, and using the following expression to measure the viscosity average molecular weight (Mv).

$$\eta sp/C = [\eta] \ (1 + 0.28 \ \eta sp)$$

$$[\eta] = 1.23 \times 10^{-4} Mv^{0.83}$$

[Molten color of bisphenol]

For the molten color of a bisphenol, 1 g of bisphenol A and 19 g of diphenyl carbonate were placed in a test tube "P-24" (2 mm$\varphi$ $\times$ 200 mm) manufactured by Nichiden-Rika Glass Co., Ltd., melted at 174°C for 30 minutes, and the Hazen color value thereof was measured by using "OME7700" manufactured by Nippon Denshoku Industries Co., Ltd. The Hazen color value obtained was multiplied by the rate of dilution with diphenyl carbonate to calculate the Hazen color value of bisphenol A.

[Measurement of pH]

The pH was measured by using a pH meter "pH METER ES-73" manufactured by HORIBA, Ltd. for an aqueous phase at 25°C taken out from a flask.

<Example 1>

[Step A-1]

(PC degradation step)

In a jacket-type separable flask equipped with a Dimroth condenser tube, a stirring blade, and a thermometer, the polycarbonate resin (80 g, the molecular weight of the repeating unit of the polycarbonate resin was 254 g/mol, and thus the number of moles of the repeating unit = 80 g / 254 g/mol = 0.315 mol), water (30 g), phenol (250 g), and 25% by mass sodium hydroxide aqueous solution (320 g) were placed at room temperature in a nitrogen atmosphere. The reaction liquid was slurry-like.
After that, the internal temperature was raised to 80°C, and the reaction was carried out for 5 hours with the internal temperature maintained at 80°C to obtain a reaction liquid a1 (uniform solution).

(Neutralization step)

After toluene (200 g) was put in the obtained reaction liquid a1, 35% by mass hydrochloric acid was added until the pH of the aqueous phase reached 8.6, and then carbon dioxide gas was generated.
After that, stirring was stopped, oil water separation was carried out, and the aqueous phase was withdrawn from the flask to obtain an organic phase a2. When the composition of part of the obtained organic phase a2 was checked by high performance liquid chromatography, bisphenol A was found to have been generated.

(Concentration step a2)

The obtained organic phase a2 was transferred to a distillation apparatus equipped with a thermometer, a stirring blade, a distilling-out tube, and a pressure regulator, and while observing the amount distilled out, the internal temperature was gradually raised to 180°C, the internal pressure was gradually lowered from normal pressure to 10 kPa to distill off water, toluene, and part of phenol, and as a result an organic phase a2-2 was obtained.

For the obtained organic phase a2-2, the pressure was restored with nitrogen, and the internal temperature was slowly lowered to 30°C to obtain a slurry a3. The obtained slurry a3 was filtered to obtain recycled bisphenol A (20 g) (crude solution A).

The time required to obtain the recycled bisphenol A (crude solution A) was 9 hours.

When the composition of part of the obtained recycled bisphenol A was checked by high performance liquid chromatography, 66.4% by mass of bisphenol A was contained. In addition, components inferred to be an undegraded portion of the polycarbonate resin and a stabilizer were contained in an amount of 0.3% by mass in terms of bisphenol A.

[Step B-1a] to [Step D-1a], [Step F-1a]

A mother liquor D was obtained according to Example 1 in the patent literature Japanese Patent Laid-Open No. 2005-220071.

The composition of the obtained mother liquor D was composed of 83% by mass of phenol, 10% by mass of bisphenol A, and 7% by mass of other components.

[Step G-1a]

The mother liquor D (100 g) and the recycled bisphenol A (20 g) (crude solution A) obtained in the step A-1 were placed in a distillation apparatus equipped with a thermometer, a stirring blade, a distilling-out tube, an oil bath, and a pressure regulator. Next, the temperature was gradually raised to 180°C, and while observing the amount distilled out, the internal pressure was gradually lowered from normal pressure to 10 kPa to distill off phenol (19 g), and as a result a mixed liquid G was obtained. After that, the pressure was restored with nitrogen.

The amount of bisphenol A contained in the mixed liquid G was 23 g (100 g × 10.0% by mass + 20 g × 66.4% by mass = 23 g).

[Step H-1a]

(Alkaline degradation/distillation step)

To the mixed liquid G, 25% by mass sodium hydroxide aqueous solution (0.4 g) was added, and evacuation was carried out to achieve full vacuum. After that, the temperature of the oil bath was raised to 230°C to obtain a fraction h1 (80 g). When the composition of part of the obtained fraction h1 was checked by high performance liquid chromatography, it was found that 93% by mass of phenol and 7% by mass of isopropenylphenol were contained. The degradation rate of bisphenol A in the alkaline degradation/distillation step was 41% (80 g × 7% by mass / 134 g/mol / 23 g × 228 g/mol × 100% = 41%). The obtained still residue was discarded.

(Rebonding step)

The cation-exchange resin A (1 g) was placed in a round-bottom flask equipped with a stirring blade, a distilling-out tube, and a water bath. In this round-bottom flask, the obtained fraction h1 (80 g) was quickly placed, and reacted at 70°C for 2 hours. After that, decantation was carried out to remove the cation-exchange resin A, giving a solution H1. When the composition of part of the obtained solution H1 was checked by high performance liquid chromatography, 11% by mass of bisphenol A was contained.

[Step I-1a], [Step B-1b]

The solution H1 (20 g), the mother liquor D (170 g), the reagent phenol (2 g), the reagent acetone (8 g), and the cation-exchange resin B (2 g) were placed in a round-bottom flask equipped with a stirring blade, a distilling-out tube, and a water bath, and reacted at 70°C for 5 hours. After that, decantation was carried out to remove the cation-exchange resin B, giving a reaction liquid B.

[Step C-1b]

The whole of the obtained reaction liquid B was transferred to a distillation apparatus equipped with a thermometer, a stirring blade, a distilling-out tube, an oil bath, and a pressure regulator, and while observing the amount distilled out, the internal temperature was gradually raised to 180°C, the internal pressure was gradually lowered from normal pressure to 10 kPa to distill off unreacted acetone, water, and part of phenol, and as a result a concentrated liquid C was obtained.

[Step D-1b]

For the obtained concentrated liquid C, the pressure was restored with nitrogen, and the internal temperature was slowly lowered to 30°C to obtain a slurry liquid. The obtained slurry liquid was filtered to obtain a cake d (11 g).

[Step E-1b]

The obtained cake d (11 g) and toluene (60 g) were placed in a separable flask equipped with a distilling-out tube and a stirring blade, and dissolved at 80°C to obtain an organic phase e1. The obtained organic phase e1 was washed 5 times with 50 g of desalinated water to obtain an organic phase e2.

The temperature of the obtained organic phase e2 was lowered to 10°C to obtain a slurry e3. The obtained slurry e3 was filtered to obtain a cake e4.

The obtained cake e4 was dried on a rotary evaporator to obtain bisphenol A (4.1 g). The Hazen color value of the obtained bisphenol A was APHA20. When the composition was checked by high performance liquid chromatography, the bisphenol A purity was 99.8% by mass.

The amount of components inferred to be an undegraded portion of the polycarbonate resin and a stabilizer was below the lower detection limit.

<Example 2>

[Step A-2]

(PC degradation step)

In a jacket-type separable flask equipped with a Dimroth condenser tube, a stirring blade, and a thermometer, the polycarbonate resin (80 g, 0.315 mol), water (30 g), phenol (240 g), and triethylamine (10 g) were placed at room temperature in a nitrogen atmosphere. The reaction liquid was slurry-like.

After that, the internal temperature was raised to 80°C, and the reaction was carried out for 5 hours with the internal temperature maintained at 80°C to obtain a reaction liquid a1 (uniform solution). During the reaction, generation of carbon dioxide was found.

When part of the obtained reaction liquid a1 was taken out and the composition was checked, the generation of bisphenol A was 19.6% by mass. The mass of the reaction liquid a1 was 80 g + 30 g + 240 g + 10 g = 360 g, the amount of the generated bisphenol was 19.6% by mass $\times$ 360 g / 228.29 g/mol = 0.309 mol, and the reaction rate was 0.309 mol / 0.315 mol $\times$ 100 = 98%.

(Concentration step a1)

The obtained reaction liquid a1 was transferred to a distillation apparatus equipped with a thermometer, a stirring blade, a distilling-out tube, and a pressure regulator, and while observing the amount distilled out, the internal temperature was gradually raised to 180°C, the internal pressure was gradually lowered from normal pressure to 10 kPa to distill off water, triethylamine, and part of phenol, and as a result a recycled bisphenol A (260 g) was obtained.

The time required to obtain the recycled bisphenol A was 7 hours.

When the composition of part of the obtained recycled bisphenol A was checked by high performance liquid chromatography, 27.0% by mass of bisphenol A was contained. In addition, components inferred to be an undegraded portion of the polycarbonate resin and a stabilizer were contained in an amount of 0.4% by mass in terms of bisphenol A.

[Step B-2a] to [Step H-2a], [Step B-2b] to [Step E-2b]

Bisphenol A (4.5 g) was obtained in the same manner as in [Step B-1a] to [Step H-1a] and [Step B-1b] to [Step E-1b] in Example 1, except that the recycled bisphenol A (50 g) obtained in the step A-2 was used instead of the recycled bisphenol A (20 g) obtained in the step A-1 in Example 1.

The Hazen color value of the obtained bisphenol A was APHA18. When the composition was checked by high performance liquid chromatography, the bisphenol A purity was 99.8% by mass.

The amount of components inferred to be an undegraded portion of the polycarbonate resin and a stabilizer was below the lower detection limit.

<Example 3>

[Step A-3]

(PC degradation step)

In a jacket-type separable flask equipped with a Dimroth condenser tube, a stirring blade, and a thermometer, the polycarbonate resin (80 g, 0.31 mol), phenol (240 g), methanol (23 g, 0.72 mol, the mole ratio to the number of moles of carbonate as the repeating unit of the polycarbonate resin: 0.72 mol / 0.31 mol = 2.3), and triethylamine (15 g, 15 g / 101 g/mol = 0.15 mol, the mole ratio to the number of moles of carbonate as the repeating unit of the polycarbonate resin: 0.15 mol / 0.31 mol = 0.48) were placed at room temperature in a nitrogen atmosphere (the amount of the liquid was 80 g + 240 g + 23 g + 15 g = 358 g).

After that, the internal temperature was raised to 85°C. An undissolved polycarbonate resin was observed in the reaction liquid when the temperature reached 85°C. The reaction was carried out for 4 hours while maintaining 85°C as it was, to obtain a uniform reaction liquid a1.

When the composition of part of the obtained reaction liquid a1 was checked by high performance liquid chromatography, bisphenol A was found to have been generated in an amount of 19.5% by mass (19.5 / 100 × 358 g / 228 g/mol / 0.31 mol = 99 mol%).

When the composition of part of the obtained reaction liquid a1 was checked by gas chromatography, dimethyl carbonate was found to have been generated in an amount of 6.5% by mass (6.5 / 100 × 358 g / 90 g/mol / 0.31 mol = 83 mol%).

(Concentration step a1)

The obtained reaction liquid a1 was transferred to a distillation apparatus equipped with a thermometer, a stirring blade, a distilling-out tube, and a pressure regulator, and while observing the amount distilled out, the internal temperature was gradually raised to 180°C, the internal pressure was gradually lowered from normal pressure to 10 kPa to distill off methanol, dimethyl carbonate, and part of phenol, and as a result a recycled bisphenol A (262 g) was obtained.

The time required to obtain the recycled bisphenol A was 7 hours.

When the composition of part of the obtained recycled bisphenol A was checked by high performance liquid chromatography, 27.1% by mass of bisphenol A was contained. In addition, components inferred to be an undegraded portion of the polycarbonate resin and a stabilizer were contained in an amount of 0.4% by mass in terms of bisphenol A.

[Step B-3a] to [Step H-3a], [Step B-3b] to [Step E-3b]

Bisphenol A (4.7 g) was obtained in the same manner as in [Step B-1a] to [Step H-1a] and [Step B-1b] to [Step E-1b] in Example 1, except that the recycled bisphenol A (50 g) obtained in the step A-3 was used instead of the recycled bisphenol A (20 g) obtained in the step A-1 in Example 1.

The Hazen color value of the obtained bisphenol A was APHA15. When the composition was checked by high performance liquid chromatography, the bisphenol A purity was 99.8% by mass.

The amount of components inferred to be an undegraded portion of the polycarbonate resin and a stabilizer was below the lower detection limit.

<Comparative Example 1>

An organic phase a2-2 was obtained in the same manner as in the PC degradation step, the neutralization step, and the concentration step a2 in the step A in Example 1.

The obtained organic phase a2-2 was washed 5 times with 50 g of desalinated water to obtain an organic phase a2-3. The temperature of the obtained organic phase a2-3 was lowered to 20°C to obtain a slurry. The obtained slurry was filtered to obtain a cake. The obtained cake was dried on a rotary evaporator to obtain bisphenol A (35 g).

The time required to obtain bisphenol A was 17 hours.

When the composition was checked by high performance liquid chromatography, the bisphenol A purity was 99.8% by mass. In addition, components inferred to be an undegraded portion of the polycarbonate resin and a stabilizer were contained in an amount of 0.1% by mass in terms of bisphenol A.

The bisphenols A obtained Examples 1 to 3 and Comparative Example 1 were compared to find that the purities of the bisphenols A were comparable. Results of visual evaluation showed that the color tones of the phenols A obtained in Examples 1 to 3 were good (colorless and transparent) in contrast to that in Comparative Example 1. While components inferred to be an undegraded portion of the polycarbonate resin and a stabilizer remained in an amount of 0.1% by mass in terms of bisphenol A in the bisphenol A obtained in Comparative Example 1, components inferred to be an undegraded portion of the polycarbonate resin and a stabilizer were not detected in the bisphenols A obtained in Examples 1 to 3, and the undegraded portion of the polycarbonate resin and stabilizer are expected to deteriorate the color tone of bisphenol A.

<Comparative Example 2>

The mother liquor D (170 g), the reagent phenol (2 g), the reagent acetone (8 g), and the cation-exchange resin B (2 g) were placed in a round-bottom flask equipped with a stirring blade, a distilling-out tube, and a water bath, and reacted at 70°C for 5 hours. After that, decantation was carried out to remove the cation-exchange resin B, giving a reaction liquid B.

The whole of the obtained reaction liquid B was transferred to a distillation apparatus equipped with a thermometer, a stirring blade, a distilling-out tube, an oil bath, and a pressure regulator, and while observing the amount distilled out, the internal temperature was gradually raised to 180°C, the internal pressure was gradually lowered from normal pressure to 10 kPa to distill off unreacted acetone, water, and part of phenol, and the pressure was restored with nitrogen to obtain concentrated liquid C (170 g).

The obtained concentrated liquid C (170 g) and the recycled bisphenol A (50 g) obtained in the step A-3 in Example 3 were placed in a round-bottom flask equipped with a stirring blade, a distilling-out tube, and a water bath, and heated to 80°C to obtain a mixed liquid. The internal temperature was slowly lowered to 30°C to obtain a slurry (crystallization step). The obtained slurry was filtered to obtain a cake d (15 g).

The obtained cake d (15 g) and toluene (60 g) were placed in a separable flask equipped with a stirring blade, a distilling-out tube, and a stirring blade, and dissolved at 80°C to obtain an organic phase e1. The obtained organic phase e1 was washed 5 times with 50 g of desalinated water to obtain an organic phase e2.

The temperature of the obtained organic phase e2 was lowered to 10°C to obtain a slurry e3. The obtained slurry e3 was filtered to obtain a cake e4.

The obtained cake e4 was dried on a rotary evaporator to obtain bisphenol A (6 g). The Hazen color value of the obtained bisphenol A was APHA89. When the composition was checked by high performance liquid chromatography, the bisphenol A purity was 99.5% by mass.

The amount of components inferred to be an undegraded portion of the polycarbonate resin and a stabilizer was 0.08% by mass.

Liquids for mixing with recycled bisphenol A (recycled BPA, crude solution A), treatments after mixing with recycled bisphenol A (recycled BPA), and the Hazen color values of obtained bisphenol A (BPA), bisphenol A (BPA) purities, and components inferred to be an undegraded portion of the polycarbonate resin (PC) and a stabilizer in Examples 1 to 3 and Comparative Example 2 are summarized in Table 1. From Table 1, it can be seen that, by mixing the recycled bisphenol A with the mother liquor D, and subjecting the mixture to the step H and returning the resultant to the step B, the Hazen color value of the bisphenol A obtained in the step E was improved and the amount of the components inferred to be an undegraded portion of the polycarbonate resin and a stabilizer reached the lower detection limit. The undegraded portion of the polycarbonate resin and component inferred to be a stabilizer are inferred to have been removed together with the still residue given in the step H.

[Table 1]

|  | Liquid for mixing with recycled BPA | Step after mixing with recycled BPA | Purity of obtained BPA | Hazen color value of obtained BPA | Components inferred to be undegraded product of PC and stabilizer |
|---|---|---|---|---|---|
| Example 1 | Mother liquor D | Step H (alkaline degradation/distillation step and rebonding step) | 99.8% by mass | APHA20 | Below lower detection limit |
| Example 2 | Mother liquor D | Step H (alkaline degradation/distillation step and rebonding step) | 99.8% by mass | APHA18 | Below lower detection limit |

(continued)

| | Liquid for mixing with recycled BPA | Step after mixing with recycled BPA | Purity of obtained BPA | Hazen color value of obtained BPA | Components inferred to be undegraded product of PC and stabilizer |
|---|---|---|---|---|---|
| Example 3 | Mother liquor D | Step H (alkaline degradation/distillation step and rebonding step) | 99.8% by mass | APHA15 | Below lower detection limit |
| Comparative Example 2 | Concentrated liquid C | Step D (crystallization step) | 99.5% by mass | APHA89 | 0.08% by mass |

<Example 4>

[Step G-4a]

The mother liquor D (18 g) obtained in Example 1 and the recycled bisphenol A (16 g) obtained in the step A-3 in Example 3 were placed in an autoclave equipped with a pressure gauge, a thermocouple, and a stirring blade to obtain a mixed liquid G.

[Step H-4a]

(Alkaline hydrolysis step)

Further, water (64 g) and sodium hydroxide (2 g) were placed in the autoclave, nitrogen was flowed therein for purging, and the autoclave was airtightly sealed. The autoclave was installed in an electric furnace with the temperature set to 250°C, and heated until the internal temperature reached 250°C. After reaching 250°C, the internal pressure was confirmed to have reached 3.7 MPa, and reaction was carried out for 2 hours. After that, the autoclave was removed from the electric furnace, and immersed in a washbowl containing ice water for 1 hour. After that, the autoclave was opened, the content was transferred to an Erlenmeyer flask, and thus a reaction liquid h2 was obtained.
When part of the reaction liquid h2 was withdrawn and the composition was checked with a gas chromatograph, 1.3% by mass of acetone was found.

(Acetone/phenol recovery step)

After neutralization by adding 2.0 g of sulfuric acid to the reaction liquid h2, the reaction liquid h2 was supplied to a distillation apparatus equipped with a thermometer, a stirring blade, a rectification column, and a water bath. Heating was carried out under normal pressure to obtain recycled acetone (0.9 g) as a first fraction and a still residue (reaction liquid h2a).
The whole of the obtained still residue was transferred to a fully jacketed separable flask equipped with a thermometer, a stirring blade, and a condenser tube, diisopropyl ether was added thereto, oil water separation was carried out at 50°C, and the aqueous phase was removed to obtain an organic phase h2b. The obtained organic phase h2b was supplied to a simple distillation apparatus equipped with a thermometer and a stirring blade. Under normal pressure, the temperature was raised to distill out and recover diisopropyl ether, and the pressure was then reduced to obtain recycled phenol (20 g).

[Step B-4b]

The mother liquor D (170 g) obtained in Example 1, the recycled phenol (2 g), the recycled acetone (0.9 g), the reagent acetone (8.1 g), and the cation-exchange resin B (2 g) were placed in a round-bottom flask equipped with a stirring blade, a distilling-out tube, and a water bath, and reacted at 70°C for 5 hours. After that, decantation was carried out to remove the cation-exchange resin B, giving a reaction liquid B.

[Step C-4b]

The whole of the obtained reaction liquid B was transferred to a distillation apparatus equipped with a thermometer, a stirring blade, a distilling-out tube, an oil bath, and a pressure regulator, and while observing the amount distilled out, the internal temperature was gradually raised to 180°C, the internal pressure was gradually lowered from normal pressure to 10 kPa to distill off unreacted acetone, water, and part of phenol, and as a result a concentrated liquid C was obtained.

[Step D-4b]

For the obtained concentrated liquid C, the pressure was restored with nitrogen, and the internal temperature was slowly lowered to 30°C to obtain a slurry. The obtained slurry was filtered to obtain a cake d (10 g).

[Step E-4b]

The obtained cake d (10 g) and toluene (60 g) were placed in a separable flask equipped with a distilling-out tube and a stirring blade, and dissolved at 80°C to obtain an organic phase e1. The obtained organic phase e1 was washed 5 times with 50 g of desalinated water to obtain an organic phase e2.

The temperature of the obtained organic phase e2 was lowered to 10°C to obtain a slurry e3. The obtained slurry e3 was filtered to obtain a cake e4.

The obtained cake e4 was dried on a rotary evaporator to obtain bisphenol A (3.2 g). The Hazen color value of the obtained bisphenol A was APHA22. When the composition was checked by high performance liquid chromatography, the bisphenol A purity was 99.8% by mass.

The amount of components inferred to be an undegraded portion of the polycarbonate resin and a stabilizer was below the lower detection limit.

<Example 5>

[Step G-5a], [Step H-5a]

In a reaction tube made of SUS316, the recycled bisphenol (1.2 g) obtained in the step A-1 in Example 1, the mother liquor D (1.3 g) obtained in the step D-1a in Example 1, and distilled water (3 g) were placed. After that, the reaction tube was airtightly sealed, and then heated to 400°C in an electric furnace, and degradation was carried out for 1 hour. After that, the reaction tube was detached from the electric furnace, and allowed to cool at room temperature. After sufficiently cooled, the reaction tube was opened, and thus a reaction liquid h4 was obtained.

This operation was repeated 10 times to obtain a reaction liquid h4 (52 g).

The obtained reaction liquid h4 was supplied to a simple distillation apparatus equipped with a stirrer and a thermometer, and while observing the amount distilled out, the internal temperature was gradually raised to 180°C, the internal pressure was gradually lowered from normal pressure to 10 kPa to distill off low-boiling-point components and water, and then phenol were distilled out to obtain recycled phenol (16 g).

[Step I-5a], [Step B-5b]

The mother liquor D (170 g), the recycled phenol (2 g), the reagent acetone (9 g), and the cation-exchange resin B (2 g) were placed in a round-bottom flask equipped with a stirring blade, a distilling-out tube, and a water bath, and reacted at 70°C for 5 hours. After that, decantation was carried out to remove the cation-exchange resin B, giving a reaction liquid B.

[Step C-5b]

The whole of the obtained reaction liquid B was transferred to a distillation apparatus equipped with a thermometer, a stirring blade, a distilling-out tube, an oil bath, and a pressure regulator, and while observing the amount distilled out, the internal temperature was gradually raised to 180°C, the internal pressure was gradually lowered from normal pressure to 10 kPa to distill off unreacted acetone, water, and part of phenol, and as a result a concentrated liquid C was obtained.

[Step D-5b]

For the obtained concentrated liquid C, the pressure was restored with nitrogen, and the internal temperature was slowly lowered to 30°C to obtain a slurry. The obtained slurry was filtered to obtain a cake d (9 g).

[Step E-5b]

The obtained cake d (9 g) and toluene (60 g) were placed in a separable flask equipped with a distilling-out tube and a stirring blade, and dissolved at 80°C to obtain an organic phase e1. The obtained organic phase e1 was washed 5 times with 50 g of desalinated water to obtain an organic phase e2.

The temperature of the obtained organic phase e2 was lowered to 10°C to obtain a slurry e3. The obtained slurry e3 was filtered to obtain a cake e4.

The obtained cake e4 was dried on a rotary evaporator to obtain bisphenol A (2.8 g). The Hazen color value of the obtained bisphenol A was APHA18. When the composition was checked by high performance liquid chromatography, the bisphenol A purity was 99.8% by mass.

The amount of components inferred to be an undegraded portion of the polycarbonate resin and a stabilizer was below the lower detection limit.

<Example 6>

[Step G-6a], [Step H-6a]

In a round-bottom flask equipped with a stirring blade, a Dimroth condenser tube, and a thermometer, the recycled bisphenol A (30 g) obtained in the step A-2 in Example 2, the mother liquor D (30 g) obtained in the step D-1a in Example 1, and p-toluenesulfonic acid (0.1 g) were placed. The round-bottom flask was immersed in an oil bath at 200°C for degradation for 3 hours. After that, the pressure was gradually reduced to obtain recycled phenol (35 g).

[Step I-6a], [Step B-6b]

The mother liquor D (170 g), the recycled phenol (2 g), the reagent acetone (9 g), and the cation-exchange resin B (2 g) were placed in a round-bottom flask equipped with a stirring blade, a distilling-out tube, and a water bath, and reacted at 70°C for 5 hours. After that, decantation was carried out to remove the cation-exchange resin B, giving a reaction liquid B.

[Step C-6b]

The whole of the obtained reaction liquid B was transferred to a distillation apparatus equipped with a thermometer, a stirring blade, a distilling-out tube, an oil bath, and a pressure regulator, and while observing the amount distilled out, the internal temperature was gradually raised to 180°C, the internal pressure was gradually lowered from normal pressure to 10 kPa to distill off unreacted acetone, water, and part of phenol, and as a result a concentrated liquid C was obtained.

[Step D-6b]

For the obtained concentrated liquid C, the pressure was restored with nitrogen, and the internal temperature was slowly lowered to 30°C to obtain a slurry liquid. The obtained slurry liquid was filtered to obtain a cake d (13 g).

[Step E-6b]

The obtained cake d (13 g) and toluene (60 g) were placed in a separable flask equipped with a distilling-out tube and a stirring blade, and dissolved at 80°C to obtain an organic phase e1. The obtained organic phase e1 was washed 5 times with 50 g of desalinated water to obtain an organic phase e2.

The temperature of the obtained organic phase e2 was lowered to 10°C to obtain a slurry e3. The obtained slurry e3 was filtered to obtain a cake e4.

The obtained cake e4 was dried on a rotary evaporator to obtain bisphenol A (3.1 g). The Hazen color value of the obtained bisphenol A was APHA15. When the composition was checked by high performance liquid chromatography, the bisphenol A purity was 99.8% by mass.

The amount of components inferred to be an undegraded portion of the polycarbonate resin and a stabilizer was below the lower detection limit.

<Example 7>

[Step H-7a]

To a distillation apparatus containing the still residue discarded in Example 1, 1 g of p-toluenesulfonic acid was added, degradation was carried out at 190°C for 2 hours, and recycled phenol (5 g) was then distilled out under full vacuum.

[Step I-7a], [Step B-7b]

The mother liquor D (170 g), the recycled phenol (2 g), the reagent acetone (9 g), and the cation-exchange resin B (2 g) were placed in a round-bottom flask equipped with a stirring blade, a distilling-out tube, and a water bath, and reacted at 70°C for 5 hours. After that, decantation was carried out to remove the cation-exchange resin B, giving a reaction liquid B.

[Step C-7b]

The whole of the obtained reaction liquid B was transferred to a distillation apparatus equipped with a thermometer, a stirring blade, a distilling-out tube, an oil bath, and a pressure regulator, and while observing the amount distilled out, the internal temperature was gradually raised to 180°C, the internal pressure was gradually lowered from normal pressure to 10 kPa to distill off unreacted acetone, water, and part of phenol, and as a result a concentrated liquid C was obtained.

[Step D-7b]

For the obtained concentrated liquid C, the pressure was restored with nitrogen, and the internal temperature was slowly lowered to 30°C to obtain a slurry. The obtained slurry was filtered to obtain a cake d (13 g).

[Step E-7b]

The obtained cake d (13 g) and toluene (60 g) were placed in a separable flask equipped with a distilling-out tube and a stirring blade, and dissolved at 80°C to obtain an organic phase e1. The obtained organic phase e1 was washed 5 times with 50 g of desalinated water to obtain an organic phase e2.
The temperature of the obtained organic phase e2 was lowered to 10°C to obtain a slurry e3. The obtained slurry e3 was filtered to obtain a cake e4.
The obtained cake e4 was dried on a rotary evaporator to obtain bisphenol A (2.7 g). The Hazen color value of the obtained bisphenol A was APHA14. When the composition was checked by high performance liquid chromatography, the bisphenol A purity was 99.8% by mass.
The amount of components inferred to be an undegraded portion of the polycarbonate resin and a stabilizer was below the lower detection limit.
Methods for degrading bisphenol A in the step H, the Hazen color values of obtained bisphenol A, bisphenol A purities, and components inferred to be an undegraded portion of the polycarbonate resin and a stabilizer in Examples 1 and 4 to 6 are summarized in Table 2. From Table 2, it can be seen that, by any of the methods for degrading bisphenol A: alkaline degradation, alkaline hydrolysis, supercritical water degradation, and acidic degradation, the Hazen color value of obtained bisphenol A was improved and the amount of components inferred to be an undegraded portion of the polycarbonate resin and a stabilizer reached below the lower detection limit.

[Table 2]

|  | Method for degrading BPA in step H | Purity of obtained BPA | Hazen color value of obtained BPA | Components inferred to be undegraded product of PC and stabilizer |
|---|---|---|---|---|
| Example 1 | Alkaline degradation | 99.8% by mass | APHA20 | Below lower detection limit |
| Example 4 | Alkaline hydrolysis | 99.8% by mass | APHA22 | Below lower detection limit |
| Example 5 | Supercritical water degradation | 99.8% by mass | APHA18 | Below lower detection limit |
| Example 6 | Acidic degradation | 99.8% by mass | APHA15 | Below lower detection limit |

<Example 8>

In a glass reaction tank having an internal volume of 45 mL equipped with a stirrer and a distilling-out tube, 10.00 g of a mixture of the bisphenols A obtained in Examples 5 to 7 (bisphenol A: 0.04 mol), 9.95 g (0.05 mol) of diphenyl carbonate, and 18 μL of 400 ppm by mass cesium carbonate aqueous solution were placed. The operation of reducing the pressure of the glass reaction tank to about 100 Pa and subsequently restoring the pressure to atmospheric pressure with nitrogen was repeated three times to purge the inside of the reaction tank with nitrogen. After that, the reaction tank was immersed in an oil bath at 220°C to dissolve the contents thereof.

The rotation speed of the stirrer was set to 100 revolutions per minute, and the pressure inside the reaction tank was reduced from 101.3 kPa to 13.3 kPa in absolute pressure over 40 minutes while distilling off phenol by-produced by the oligomerization reaction of bisphenol A and diphenyl carbonate in the reaction tank.

Next, an ester exchange reaction was carried out for 80 minutes while holding the pressure inside the reaction tank at 13.3 kPa and further distilling off phenol.

After that, the temperature outside the reaction tank was raised to 290°C, and the pressure inside the reaction tank was reduced from 13.3 kPa to 399 Pa in absolute pressure over 40 minutes to remove the distilled-out phenol outside the system.

After that, the absolute pressure of the reaction tank was reduced to 30 Pa, and a polycondensation reaction was carried out. The polycondensation reaction was completed when the stirrer in the reaction tank had a predetermined stirring power. The time from the temperature raising to 290°C to the completion of the polymerization was 120 minutes.

Next, the pressure of the reaction tank was restored to 101.3 kPa in absolute pressure with nitrogen, then the pressure was increased to 0.2 MPa in gauge pressure, and the polycarbonate resin was withdrawn from the reaction tank to obtain a polycarbonate resin. The viscosity average molecular weight (Mv) of the obtained polycarbonate resin was 26500.

<Example 9>

A mother liquor D and recycled bisphenol A (crude solution A) were obtained in the same manner as in Example 1. The obtained mother liquor D (100 g) was placed in a jacket-type separable flask equipped with a Dimroth condenser tube, a stirring blade, and a thermometer with the temperature kept at 80°C. The obtained recycled bisphenol A (20 g) was placed in an eggplant-shaped flask, which was immersed in an oil bath at 120°C for melting. A glass funnel having an inner diameter of 5 mm was used for placing the melted recycled bisphenol A in the jacket-type separable flask, and the melted recycled bisphenol A was successfully supplied thereto without clogging, giving a uniform solution. The obtained uniform solution was placed in a distillation apparatus equipped with a thermometer, a stirring blade, a distilling-out tube, an oil bath, and a pressure regulator.

Next, the temperature was gradually raised to 180°C, and while observing the amount distilled out, the internal pressure was gradually lowered from normal pressure to 10 kPa to distill off phenol (19 g), and as a result a mixed liquid G was obtained. After that, the pressure was restored with nitrogen.

The amount of bisphenol A contained in the mixed liquid G was 23 g (100 g × 10.0% by mass + 20 g × 66.4% by mass = 23 g).

(Alkaline degradation/distillation step)

To the mixed liquid G, 25% by mass sodium hydroxide aqueous solution (0.4 g) was added, and evacuation was carried out to achieve full vacuum. After that, the temperature of the oil bath was raised to 230°C to obtain a fraction h1 (80 g). When the composition of part of the obtained fraction h1 was checked by high performance liquid chromatography, it was found that 93% by mass of phenol and 7% by mass of isopropenylphenol were contained. The degradation rate of bisphenol A in the alkaline degradation/distillation step was 41% (80 g × 7% by mass / 134 g/mol / 23 g × 228 g/mol × 100% = 41%). The obtained still residue was discarded.

<Comparative Example 3>

A mother liquor D was obtained in the same manner as in Example 1. Bisphenol A was obtained in the same manner as in Comparative Example 1. The mother liquor D (100 g) was placed in a jacket-type separable flask provided with a Dimroth condenser tube, a stirring blade, and a thermometer with the temperature kept at 80°C. The bisphenol A (20 g) was placed in an eggplant-shaped flask, which was immersed in an oil bath at 120°C, but the bisphenol A was not successfully melted because of the high purity. Hence, the bisphenol A was not successfully supplied to a distillation apparatus equipped with a thermometer, a stirring blade, a distilling-out tube, an oil bath, and a pressure regulator by using a glass funnel having an inner diameter of 5 mm.

<Comparative Example 4>

A mother liquor D was obtained in the same manner as in Example 1. Bisphenol A was obtained in the same manner as in Comparative Example 1. The mother liquor D (100 g) was placed in a jacket-type separable flask equipped with a Dimroth condenser tube, a stirring blade, and a thermometer. The obtained bisphenol A (20 g) was placed in an eggplant-shaped flask, which was immersed in an oil bath at 180°C for melting. A glass funnel having an inner diameter of 5 mm was used for placing the melted recycled bisphenol A in the jacket-type separable flask, and the melted bisphenol A was successfully supplied thereto without clogging, giving a uniform solution. The obtained uniform solution was significantly colored in reddish brown because of the high melting temperature.

The obtained uniform solution was placed in a distillation apparatus equipped with a thermometer, a stirring blade, a distilling-out tube, an oil bath, and a pressure regulator.

Next, the temperature was gradually raised to 180°C, and while observing the amount distilled out, the internal pressure was gradually lowered from normal pressure to 10 kPa to distill off phenol (19 g), and as a result a mixed liquid G was obtained. After that, the pressure was restored with nitrogen.

The amount of bisphenol A contained in the mixed liquid G was 30 g (100 g $\times$ 10.0% by mass + 20 g $\times$ 99.8% by mass = 30 g).

(Alkaline degradation/distillation step)

To the mixed liquid G, 25% by mass sodium hydroxide aqueous solution (0.6 g) was added, and evacuation was carried out to achieve full vacuum. After that, the temperature of the oil bath was raised to 230°C to obtain a fraction h1 (50 g). When the composition of part of the obtained fraction h1 was checked by high performance liquid chromatography, it was found that 97% by mass of phenol and 3% by mass of isopropenylphenol were contained. The degradation rate of bisphenol A in the alkaline degradation/distillation step was 14% (80 g $\times$ 3% by mass / 134 g/mol / 30 g $\times$ 228 g/mol $\times$ 100% = 14%). The obtained still residue was discarded.

Bisphenol A purities of recycled bisphenol A (BPA contents of crude solution A), melting temperatures for recycled bisphenol A, success or failure in supplying by using a glass funnel having an inner diameter of 5 mm, and degradation rates of bisphenol A in the alkaline degradation/distillation step in Example 9, Comparative Example 3, and Comparative Example 4 are summarized in Table 3. From Table 3, it can be seen that high bisphenol A purity of recycled bisphenol A makes it impossible to supply recycled bisphenol A at low melting temperature. It can also be seen that higher melting temperature set for supplying recycled bisphenol A results in significantly deteriorated and altered color tone, and thus the lowering of the degradation rate of bisphenol A in the alkaline degradation/distillation step.

[Table 3]

| | Purity of recycled bisphenol A | Melting temperature for recycled bisphenol A | Success or failure in supplying by using glass funnel having inner diameter of 5 mm | Degradation rate of bisphenol A in alkaline degradation/distillation step |
|---|---|---|---|---|
| Example 9 | 66.40% | 120°C | Succeeded | 41% |
| Comparative Example 3 | 99.80% | 120°C | Failed | - |
| Comparative Example 4 | 99.80% | 180°C | Succeeded | 14% |

<Comparative Example 5>

In a 200 mL autoclave equipped with an induction stirring blade, a pressure gauge, and a thermometer, 30 g (30 g / 254 g/mol = 0.12 mol) of the polycarbonate resin, 100 g of phenol, and 1 g of sodium carbonate were placed. After purging three times with nitrogen, the autoclave was installed in an electric furnace, and reaction was carried out at an internal temperature of 250°C for 3 hours. After the reaction, the autoclave was immersed in ice water, and the internal pressure was restored to normal pressure to obtain a reaction liquid.

The obtained reaction liquid was placed in a distillation apparatus equipped with a thermometer, a stirring blade, a distilling-out tube, an oil bath, and a pressure regulator.

Next, the temperature was gradually raised to 180°C, and while observing the amount distilled out, the internal pressure was gradually lowered from normal pressure to 10 kPa to distill off phenol (100 g), and as a result a concentrated liquid was obtained.

(Alkaline degradation/distillation step)

Evacuation was carried out to achieve full vacuum in the distillation apparatus. After that, the temperature of the oil bath was raised to 230°C to obtain a fraction h1 (5 g). When the composition of part of the obtained fraction h1 was checked by high performance liquid chromatography, the fraction h1 was mostly phenol, and isopropenylphenol was not detected.

Industrial Applicability

According to the present invention, bisphenol A can be produced from a polycarbonate resin contained in a waste plastic or the like, and obtained bisphenol A can be used as a raw material, a curing agent, or an additive for resins including polycarbonate resins, which is industrially useful.

**Claims**

1.   A method for producing bisphenol A, comprising the following step A to step F, step H, and step I:

    step A: a step of degrading a polycarbonate resin in a solvent to obtain a reaction liquid a1 containing bisphenol A, and distilling off the solvent from the obtained reaction liquid a1 to obtain a crude solution A having a bisphenol A content of less than 90% by mass;
    step B: a step of subjecting acetone and phenol to dehydration condensation in the presence of an acid catalyst to obtain a reaction liquid B containing bisphenol A;
    step C: a step of distilling off unreacted acetone and water from the reaction liquid B obtained in the step B to obtain a concentrated liquid C;
    step D: a step of subjecting the concentrated liquid C obtained in the step C to crystallization to obtain a slurry liquid, and subjecting the slurry liquid to solid-liquid separation to obtain a mother liquor D and a cake d;
    step E: a step of purifying the cake d obtained in the step D to obtain bisphenol A;
    step F: a step of circulating part of the mother liquor D obtained in the step D to supply to the step B;
    step H: a step of obtaining a solution H1 or a solution H2 from the crude solution A and part of the mother liquor D, wherein

       the solution H1 is a solution containing bisphenol A obtained by degrading bisphenol A contained in the crude solution A and the mother liquor D into phenol and isopropenylphenol under conditions that allow degradation of bisphenol A and then rebonding phenol and isopropenylphenol, and
       the solution H2 is a solution containing phenol obtained by degrading bisphenol A contained in the crude solution A and the mother liquor D into phenol and acetone under conditions that allow degradation of bisphenol A; and

    step I: a step of supplying the solution H1 or the solution H2 obtained in the step H to the step B and/or the step C.

2.   A method for producing bisphenol A, comprising the following step A to step I:

    step A: a step of degrading a polycarbonate resin in a solvent to obtain a reaction liquid a1 containing bisphenol A, and distilling off the solvent from the obtained reaction liquid a1 to obtain a crude solution A having a bisphenol A content of less than 90% by mass;
    step B: a step of subjecting acetone and phenol to dehydration condensation in the presence of an acid catalyst to obtain a reaction liquid B containing bisphenol A;
    step C: a step of distilling off unreacted acetone and water from the reaction liquid B obtained in the step B to obtain a concentrated liquid C;
    step D: a step of subjecting the concentrated liquid C obtained in the step C to crystallization to obtain a slurry liquid, and subjecting the slurry liquid to solid-liquid separation to obtain a mother liquor D and a cake d;
    step E: a step of purifying the cake d obtained in the step D to obtain bisphenol A;
    step F: a step of circulating part of the mother liquor D obtained in the step D to supply to the dehydration condensation in the step B;
    step G: a step of mixing the crude solution A obtained in the step A and part of the mother liquor D obtained in the step D to obtain a mixed liquid G;
    step H: a step of obtaining a solution H1 containing bisphenol A, wherein the solution H1 is obtained by treating the mixed liquid G under conditions that allow degradation of bisphenol A and then rebonding, or a solution H2 containing a degradation product,

wherein the solution H2 is obtained by treating the mixed liquid G under conditions that allow degradation of bisphenol A; and

step I: a step of supplying the solution H1 or the solution H2 obtained in the step H to the step B and/or the step C.

3. The method for producing bisphenol A according to claim 1 or 2, wherein the crude solution A and part of the mother liquor D are mixed, and a mixed liquid G obtained is supplied to an apparatus that carries out degradation of bisphenol A in the step H.

4. The method for producing bisphenol A according to claim 1 or 2, wherein the crude solution A and part of the mother liquor D are each supplied to an apparatus that carries out degradation of bisphenol A in the step H, and degradation reaction is carried out while a mixed liquid G is prepared in the apparatus.

5. The method for producing bisphenol A according to any one of claims 1 to 4, wherein, in the step C, part of phenol is further distilled off from the reaction liquid B to obtain a concentrated liquid C.

6. The method for producing bisphenol A according to any one of claims 2 to 5, wherein, in the step H, a degradation rate of bisphenol A in the mixed liquid G after treating the mixed liquid G under the conditions that allow degradation of bisphenol A is 30 mol% or more.

7. The method for producing bisphenol A according to any one of claims 1 to 6, wherein the conditions that allow degradation of bisphenol A are any of alkaline conditions, acidic conditions, and supercritical water conditions.

8. The method for producing bisphenol A according to any one of claims 2 to 7, wherein distillation is carried out after or while the mixed liquid G is treated under conditions that allow degradation of bisphenol A, a fraction h containing a degradation product is recovered, and a residue is removed.

9. The method for producing bisphenol A according to any one of claims 2 to 8, wherein the step H is a step of obtaining the solution H1, and includes:

a degradation/distillation step of carrying out distillation while the mixed liquid G is treated under alkaline conditions that allow degradation of bisphenol A, recovering a fraction h1 containing phenol and isopropenylphenol as a degradation product, and removing a residue; and

a rebonding step of rebonding phenol and isopropenylphenol contained in the fraction h1 to generate bisphenol A.

10. The method for producing bisphenol A according to claim 9, wherein the fraction h1 contains 1.0% by mass or more of isopropenylphenol.

11. The method for producing bisphenol A according to claim 9 or 10, wherein the solution H1 contains 1% by mass or more of bisphenol A.

12. The method for producing bisphenol A according to any one of claims 9 to 11, wherein distillation is carried out after the residue is treated in the presence of an acid catalyst and a phenol fraction h1b is recovered.

13. The method for producing bisphenol A according to any one of claims 2 to 8, wherein the step H is a step of obtaining the solution H2, and includes:

an alkaline hydrolysis step of treating the mixed liquid G under alkaline conditions that allow hydrolysis of bisphenol A to obtain a reaction liquid h2 containing acetone and phenol; and

an acetone/phenol recovery step of recovering an acetone fraction and/or a phenol fraction from the reaction liquid h2 obtained in the alkaline hydrolysis step and removing a residue.

14. The method for producing bisphenol A according to claim 13, wherein the solution H2 contains 0.1% by mass or more of acetone.

15. The method for producing bisphenol A according to any one of claims 1 to 14, wherein the solvent to be used in the step A is phenol.

EP 4 269 379 B1

16. The method for producing bisphenol A according to any one of claims 1 to 15, wherein the step A is a step of degrading the polycarbonate resin in the presence of any catalyst selected from the group consisting of an alkali metal hydroxide, an alkali metal carbonate, an alkylamine, and an acid to obtain the crude solution A.

17. The method for producing bisphenol A according to any one of claims 1 to 16, wherein a bisphenol A content of the crude solution A is 10% by mass or more.

18. The method for producing bisphenol A according to claim 17, wherein a bisphenol A content of the crude solution A is 20% by mass or more.

19. A method for producing a polycarbonate resin, comprising producing bisphenol A according to any one of claims 1 to 18 and producing a polycarbonate resin from the bisphenol A.

**Patentansprüche**

1. Ein Verfahren zur Herstellung von Bisphenol A, umfassend den folgenden Schritt A bis Schritt F, Schritt H und Schritt I:

Schritt A: einen Schritt des Abbaus eines Polycarbonatharzes in einem Lösungsmittel, um eine Bisphenol A enthaltende Reaktionsflüssigkeit a1 zu erhalten, und des Abdestillierens des Lösungsmittels aus der erhaltenen Reaktionsflüssigkeit a1, um eine Rohlösung A mit einem Bisphenol-A-Gehalt von weniger als 90 Massen-% zu erhalten;
Schritt B: einen Schritt, bei dem Aceton und Phenol einer Dehydrierungskondensation in Gegenwart eines Säurekatalysators unterzogen werden, um eine Bisphenol A enthaltende Reaktionsflüssigkeit B zu erhalten;
Schritt C: einen Schritt des Abdestillierens von nicht umgesetztem Aceton und Wasser aus der im Schritt B erhaltenen Reaktionsflüssigkeit B, um eine konzentrierte Flüssigkeit C zu erhalten;
Schritt D: einen Schritt, bei dem die im Schritt C erhaltene konzentrierte Flüssigkeit C einer Kristallisation unterzogen wird, um eine Aufschlämmungsflüssigkeit zu erhalten, und bei dem die Aufschlämmungsflüssigkeit einer Fest-Flüssig-Trennung unterzogen wird, um eine Mutterlauge D und einen Kuchen d zu erhalten;
Schritt E: einen Schritt des Reinigens des im Schritt D erhaltenen Kuchens d, um Bisphenol A zu erhalten;
Schritt F: einen Schritt des Zirkulierens eines Teils der im Schritt D erhaltenen Mutterlauge D, um sie dem Schritt B zuzuführen;
Schritt H: einen Schritt des Erhaltens einer Lösung H1 oder einer Lösung H2 aus der Rohlösung A und einem Teil der Mutterlauge D, wobei

die Lösung H1 eine Lösung ist, die Bisphenol A enthält, das durch Abbau von in der Rohlösung A und der Mutterlauge D enthaltenem Bisphenol A zu Phenol und Isopropenylphenol unter Bedingungen, die einen Abbau von Bisphenol A ermöglichen, und anschließende erneute Bindung von Phenol und Isopropenylphenol erhalten wird, und
die Lösung H2 eine Lösung ist, die Phenol enthält, das durch Abbau von in der Rohlösung A und der Mutterlauge D enthaltenem Bisphenol A zu Phenol und Aceton unter Bedingungen, die einen Abbau von Bisphenol A ermöglichen, erhalten wird; und

Schritt I: einen Schritt der Zufuhr der im Schritt H erhaltenen Lösung H1 oder Lösung H2 zum Schritt B und/oder dem Schritt C.

2. Ein Verfahren zur Herstellung von Bisphenol A, umfassend den folgenden Schritt A bis Schritt I:

Schritt A: einen Schritt des Abbaus eines Polycarbonatharzes in einem Lösungsmittel, um eine Bisphenol A enthaltende Reaktionsflüssigkeit a1 zu erhalten, und des Abdestillierens des Lösungsmittels aus der erhaltenen Reaktionsflüssigkeit a1, um eine Rohlösung A mit einem Bisphenol A-Gehalt von weniger als 90 Massen-% zu erhalten;
Schritt B: einen Schritt, bei dem Aceton und Phenol einer Dehydrierungskondensation in Gegenwart eines Säurekatalysators unterzogen werden, um eine Bisphenol A enthaltende Reaktionsflüssigkeit B zu erhalten;
Schritt C: einen Schritt des Abdestillierens von nicht umgesetztem Aceton und Wasser aus der im Schritt B erhaltenen Reaktionsflüssigkeit B, um eine konzentrierte Flüssigkeit C zu erhalten;
Schritt D: einen Schritt, bei dem die im Schritt C erhaltene konzentrierte Flüssigkeit C einer Kristallisation unterzogen wird, um eine Aufschlämmungsflüssigkeit zu erhalten, und bei dem die Aufschlämmungsflüssigkeit einer Fest-Flüssig-Trennung unterzogen wird, um eine Mutterlauge D und einen Kuchen d zu erhalten;

Schritt E: einen Schritt des Reinigens des im Schritt D erhaltenen Kuchens d, um Bisphenol A zu erhalten;

Schritt F: einen Schritt des Zirkulierens eines Teils der im Schritt D erhaltenen Mutterlauge D, um sie der Dehydrierungskondensation im Schritt B zuzuführen;

Schritt G: einen Schritt des Mischens der im Schritt A erhaltenen Rohlösung A und eines Teils der im Schritt D erhaltenen Mutterlauge D, um ein Flüssigkeitsgemisch G zu erhalten;

Schritt H: einen Schritt des Erhaltens einer Lösung H1, die Bisphenol A enthält, wobei die Lösung H1 durch Behandeln des Flüssigkeitsgemischs G unter Bedingungen, die einen Abbau von Bisphenol A ermöglichen, und anschließende erneute Bindung erhalten wird, oder einer Lösung H2, die ein Abbauprodukt enthält, wobei die Lösung H2 durch Behandeln des Flüssigkeitsgemischs G unter Bedingungen, die einen Abbau von Bisphenol A ermöglichen, erhalten wird; und

Schritt I: einen Schritt der Zufuhr der im Schritt H erhaltenen Lösung H1 oder Lösung H2 zum Schritt B und/oder dem Schritt C.

3. Das Verfahren zur Herstellung von Bisphenol A nach Anspruch 1 oder 2, wobei die Rohlösung A und ein Teil der Mutterlauge D gemischt werden und ein erhaltenes Flüssigkeitsgemisch G einer Vorrichtung zugeführt wird, die den Abbau von Bisphenol A im Schritt H durchführt.

4. Das Verfahren zur Herstellung von Bisphenol A nach Anspruch 1 oder 2, wobei die Rohlösung A und ein Teil der Mutterlauge D jeweils einer Vorrichtung zugeführt werden, die den Abbau von Bisphenol A im Schritt H durchführt, und die Abbaureaktion durchgeführt wird, während in der Vorrichtung ein Flüssigkeitsgemisch G hergestellt wird.

5. Das Verfahren zur Herstellung von Bisphenol A nach einem der Ansprüche 1 bis 4, wobei im Schritt C ein Teil des Phenols aus der Reaktionsflüssigkeit B weiter abdestilliert wird, um eine konzentrierte Flüssigkeit C zu erhalten.

6. Das Verfahren zur Herstellung von Bisphenol A nach einem der Ansprüche 2 bis 5, wobei im Schritt H eine Abbaurate von Bisphenol A im Flüssigkeitsgemisch G nach der Behandlung des Flüssigkeitsgemischs G unter den Bedingungen, die einen Abbau von Bisphenol A ermöglichen, 30 mol-% oder mehr beträgt.

7. Das Verfahren zur Herstellung von Bisphenol A nach einem der Ansprüche 1 bis 6, wobei die Bedingungen, die einen Abbau von Bisphenol A ermöglichen, irgendwelche aus alkalischen Bedingungen, sauren Bedingungen und überkritischen Wasserbedingungen sind.

8. Das Verfahren zur Herstellung von Bisphenol A nach einem der Ansprüche 2 bis 7, wobei die Destillation durchgeführt wird, nachdem oder während das Flüssigkeitsgemisch G unter Bedingungen behandelt wird, die einen Abbau von Bisphenol A ermöglichen, eine Fraktion h, die ein Abbauprodukt enthält, gewonnen wird und ein Rückstand entfernt wird.

9. Das Verfahren zur Herstellung von Bisphenol A nach einem der Ansprüche 2 bis 8, wobei der Schritt H ein Schritt des Erhaltens der Lösung H1 ist und beinhaltet:

einen Abbau-/Destillationsschritt, bei dem eine Destillation durchgeführt wird, während das Flüssigkeitsgemisch G unter alkalischen Bedingungen, die den Abbau von Bisphenol A ermöglichen, behandelt wird, eine Fraktion h1 rückgewonnen wird, die Phenol und Isopropenylphenol als ein Abbauprodukt enthält, und ein Rückstand entfernt wird; und

einen Schritt des erneuten Bindens von Phenol und Isopropenylphenol, die in der Fraktion h1 enthalten sind, um Bisphenol A zu erzeugen.

10. Das Verfahren zur Herstellung von Bisphenol A nach Anspruch 9, wobei die Fraktion h1 1,0 Massen-% oder mehr Isopropenylphenol enthält.

11. Das Verfahren zur Herstellung von Bisphenol A nach Anspruch 9 oder 10, wobei die Lösung H1 1 Massen-% oder mehr Bisphenol A enthält.

12. Das Verfahren zur Herstellung von Bisphenol A nach einem der Ansprüche 9 bis 11, wobei eine Destillation durchgeführt wird, nachdem der Rückstand in Gegenwart eines Säurekatalysators behandelt wurde, und eine Phenolfraktion h1b gewonnen wird.

13. Das Verfahren zur Herstellung von Bisphenol A nach einem der Ansprüche 2 bis 8, wobei der Schritt H ein Schritt des Erhaltens der Lösung H2 ist und beinhaltet:

einen alkalischen Hydrolyseschritt bei dem das Flüssigkeitsgemisch G unter alkalischen Bedingungen behandelt wird, die eine Hydrolyse von Bisphenol A ermöglichen, um eine Reaktionsflüssigkeit h2 zu erhalten, die Aceton und Phenol enthält; und
einen Aceton/Phenol-Rückgewinnungsschritt, bei dem eine Acetonfraktion und/oder einer Phenolfraktion aus der in dem alkalischen Hydrolyseschritt erhaltenen Reaktionsflüssigkeit h2 rückgewonnen wird und ein Rückstand entfernt wird.

14. Das Verfahren zur Herstellung von Bisphenol A nach Anspruch 13, wobei die Lösung H2 0,1 Massen-% oder mehr Aceton enthält.

15. Das Verfahren zur Herstellung von Bisphenol A nach einem der Ansprüche 1 bis 14, wobei das in dem Schritt A zu verwendende Lösungsmittel Phenol ist.

16. Das Verfahren zur Herstellung von Bisphenol A nach einem der Ansprüche 1 bis 15, wobei der Schritt A ein Schritt des Abbaus des Polycarbonatharzes in Gegenwart eines beliebigen Katalysators, ausgewählt aus der Gruppe bestehend aus einem Alkalimetallhydroxid, einem Alkalimetallcarbonat, einem Alkylamin und einer Säure, ist, um die Rohlösung A zu erhalten.

17. Das Verfahren zur Herstellung von Bisphenol A nach einem der Ansprüche 1 bis 16, wobei ein Bisphenol-A-Gehalt der Rohlösung A 10 Massen-% oder mehr beträgt.

18. Das Verfahren zur Herstellung von Bisphenol A nach Anspruch 17, wobei ein Bisphenol A-Gehalt der Rohlösung A 20 Massen-% oder mehr beträgt.

19. Ein Verfahren zur Herstellung eines Polycarbonatharzes, umfassend die Herstellung von Bisphenol A nach einem der Ansprüche 1 bis 18 und die Herstellung eines Polycarbonatharzes aus dem Bisphenol A.

**Revendications**

1. Méthode pour produire du bisphénol A, comprenant l'étape A à l'étape F, l'étape H, et l'étape I qui suivent :

étape A : une étape de dégradation d'une résine de polycarbonate dans un solvant pour que soit obtenu un liquide réactionnel a1 contenant du bisphénol A, et d'élimination du solvant par distillation du liquide réactionnel a1 obtenu pour que soit obtenue une solution brute A ayant une teneur en bisphénol A inférieure à 90 % en masse ;
étape B : une étape de soumission d'acétone et de phénol à une condensation par déshydratation en présence d'un catalyseur acide pour que soit obtenu un liquide réactionnel B contenant du bisphénol A ;
étape C : une étape d'élimination de l'acétone n'ayant pas réagi et de l'eau par distillation du liquide réactionnel B obtenu dans l'étape B pour que soit obtenu un liquide concentré C ;
étape D : une étape de soumission du liquide concentré C obtenu dans l'étape C à une cristallisation pour que soit obtenue une bouillie liquide, et de soumission de la bouillie liquide à une séparation solide-liquide pour que soient obtenus une liqueur mère D et un gâteau d ;
étape E : une étape de purification du gâteau d obtenu dans l'étape D pour que soit obtenu du bisphénol A ;
étape F : une étape de circulation d'une partie de la liqueur mère D obtenue dans l'étape D pour qu'elle soit fournie à l'étape B ;
étape H : une étape d'obtention d'une solution H1 ou d'une solution H2 à partir de la solution brute A et d'une partie de la liqueur mère D, dans laquelle

la solution H1 est une solution contenant du bisphénol A obtenu par dégradation de bisphénol A contenu dans la solution brute A et la liqueur mère D en phénol et isopropénylphénol dans des conditions qui permettent la dégradation de bisphénol A et ensuite re-liaison du phénol et de l'isopropénylphénol, et
la solution H2 est une solution contenant du phénol obtenu par dégradation de bisphénol A contenu dans la solution brute A et la liqueur mère D en phénol et acétone dans des conditions qui permettent la dégradation de bisphénol A ; et

étape I : une étape de fourniture de la solution H1 ou de la solution H2 obtenue dans l'étape H à l'étape B et/ou à l'étape C.

2. Méthode pour produire du bisphénol A, comprenant les étapes A à I suivantes :

étape A : une étape de dégradation d'une résine de polycarbonate dans un solvant pour que soit obtenu un liquide réactionnel a1 contenant du bisphénol A, et d'élimination du solvant par distillation du liquide réactionnel a1 obtenu pour que soit obtenue une solution brute A ayant une teneur en bisphénol A inférieure à 90 % en masse ;
étape B : une étape de soumission d'acétone et de phénol à une condensation par déshydratation en présence d'un catalyseur acide pour que soit obtenu un liquide réactionnel B contenant du bisphénol A ;
étape C : une étape d'élimination de l'acétone n'ayant pas réagi et de l'eau par distillation du liquide réactionnel B obtenu dans l'étape B pour que soit obtenu un liquide concentré C ;
étape D : une étape de soumission du liquide concentré C obtenu dans l'étape C à une cristallisation pour que soit obtenue une bouillie liquide, et de soumission de la bouillie liquide à une séparation solide-liquide pour que soient obtenus une liqueur mère D et un gâteau d ;
étape E : une étape de purification du gâteau d obtenu dans l'étape D pour que soit obtenu du bisphénol A ;
étape F : une étape de circulation d'une partie de la liqueur mère D obtenue dans l'étape D pour qu'elle soit fournie à la condensation par déshydratation dans l'étape B ;
étape G : une étape de mélange de la solution brute A obtenue dans l'étape A et d'une partie de la liqueur mère D obtenue dans l'étape D pour que soit obtenu un liquide mixte G ;
étape H : une étape d'obtention d'une solution H1 contenant du bisphénol A, dans laquelle la solution H1 est obtenue par traitement du liquide mixte G dans des conditions qui permettent la dégradation de bisphénol A et ensuite re-liaison, ou d'une solution H2 contenant un produit de dégradation, dans laquelle la solution H2 est obtenue par traitement du liquide mixte G dans des conditions qui permettent la dégradation de bisphénol A ; et
étape I : une étape de fourniture de la solution H1 ou de la solution H2 obtenue dans l'étape H à l'étape B et/ou à l'étape C.

3. Méthode pour produire du bisphénol A selon la revendication 1 ou 2, dans laquelle la solution brute A et une partie de la liqueur mère D sont mélangées, et un liquide mixte G obtenu est fourni à un appareil qui effectue la dégradation de bisphénol A dans l'étape H.

4. Méthode pour produire du bisphénol A selon la revendication 1 ou 2, dans laquelle la solution brute A et une partie de la liqueur mère D sont fournies chacune à un appareil qui effectue la dégradation du bisphénol A dans l'étape H, et une réaction de dégradation est effectuée tandis qu'un liquide mixte G est préparé dans l'appareil.

5. Méthode pour produire du bisphénol A selon l'une quelconque des revendications 1 à 4, dans laquelle, dans l'étape C, une partie du phénol est en outre éliminée par distillation du liquide réactionnel B pour que soit obtenu un liquide concentré C.

6. Méthode pour produire du bisphénol A selon l'une quelconque des revendications 2 à 5, dans laquelle, dans l'étape H, le taux de dégradation du bisphénol A dans le liquide mixte G après traitement du liquide mixte G dans des conditions qui permettent la dégradation de bisphénol A est de 30 % en moles ou plus.

7. Méthode pour produire du bisphénol A selon l'une quelconque des revendications 1 à 6, dans laquelle les conditions qui permettent la dégradation de bisphénol A sont n'importe lesquelles parmi des conditions alcalines, des conditions acides, et des conditions aqueuses supercritiques.

8. Méthode pour produire du bisphénol A selon l'une quelconque des revendications 2 à 7, dans laquelle la distillation est effectuée après ou pendant que le liquide mixte G est traité dans des conditions qui permettent la dégradation de bisphénol A, une fraction h contenant un produit de dégradation est récupéré, et un résidu est retiré.

9. Méthode pour produire du bisphénol A selon l'une quelconque des revendications 2 à 8, dans laquelle l'étape H est une étape d'obtention de la solution H1, et comprend :

une étape de dégradation/distillation dans laquelle une distillation est effectuée alors que le liquide mixte G est traité dans des conditions alcalines qui permettent la dégradation de bisphénol A, une fraction h1 contenant du phénol et de l'isopropénylphénol en tant que produit de dégradation est récupérée, et un résidu est retiré ; et
une étape de re-liaison dans laquelle le phénol et l'isopropénylphénol contenus dans la fraction h1 sont re-liés pour générer du bisphénol A.

10. Méthode pour produire du bisphénol A selon la revendication 9, dans laquelle la fraction h1 contient 1,0 % en masse ou plus d'isopropénylphénol.

11. Méthode pour produire du bisphénol A selon la revendication 9 ou 10, dans laquelle la solution H1 contient 1 % en masse ou plus de bisphénol A.

12. Méthode pour produire du bisphénol A selon l'une quelconque des revendications 9 à 11, dans laquelle la distillation est effectuée après que le résidu a été traité en présence d'un catalyseur acide, et une fraction de phénol h1b est récupérée.

13. Méthode pour produire du bisphénol A selon l'une quelconque des revendications 2 à 8, dans laquelle l'étape H est une étape d'obtention de la solution H2, et comprend :

   une étape d'hydrolyse alcaline dans laquelle le liquide mixte G est traité dans des conditions alcalines qui permettent l'hydrolyse de bisphénol A pour que soit obtenu un liquide réactionnel h2 contenant de l'acétone et du phénol ; et
   une étape de récupération d'acétone/phénol dans laquelle une fraction d'acétone et/ou une fraction de phénol sont récupérées à partir du liquide réactionnel h2 obtenu dans l'étape d'hydrolyse alcaline et un résidu est retiré.

14. Méthode pour produire du bisphénol A selon la revendication 13, dans laquelle la solution H2 contient 0,1 % en masse ou plus d'acétone.

15. Méthode pour produire du bisphénol A selon l'une quelconque des revendications 1 à 14, dans laquelle le solvant devant être utilisé dans l'étape A est le phénol.

16. Méthode pour produire du bisphénol A selon l'une quelconque des revendications 1 à 15, dans laquelle l'étape A est une étape de dégradation de la résine de polycarbonate en présence d'un catalyseur quelconque choisi dans le groupe constitué par un hydroxyde de métal alcalin, un carbonate de métal alcalin, une alkylamine, et un acide, pour que soit obtenue la solution brute A.

17. Méthode pour produire du bisphénol A selon l'une quelconque des revendications 1 à 16, dans laquelle la teneur en bisphénol A de la solution brute A est de 10 % en masse ou plus.

18. Méthode pour produire du bisphénol A selon la revendication 17, dans laquelle la teneur en bisphénol A de la solution brute est de 20 % en masse ou plus.

19. Méthode pour produire une résine de polycarbonate, comprenant la production de bisphénol A selon l'une quelconque des revendications 1 à 18 et la production d'une résine de polycarbonate à partir du bisphénol A.

Fig. 1

EP 4 269 379 B1

# Fig. 2

Fig. 3

Step A1

Fig. 4

Fig. 5

Fig. 6

Step H1

## Fig. 7

Step H1a

## Fig. 8

Step H2

Fig. 9

Fig. 10

Step H4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005112781 A
- JP 2006036668 A
- US 5198591 A
- JP 63132850 A
- JP 2028126 A
- JP 2012201619 A
- WO 2012108385 A
- JP 2005220071 A